(19) Europäisches Patentamt
European Patent Office
Office européen des brevets

(11) Veröffentlichungsnummer : **0 132 225**
**B1**

(12) # EUROPÄISCHE PATENTSCHRIFT

(45) Veröffentlichungstag der Patentschrift : **19.08.87**

(51) Int. Cl.$^4$ : **C 08 K 5/42**

(21) Anmeldenummer : **84810311.5**

(22) Anmeldetag : **25.06.84**

(54) **Härtung von säurehärtbaren Zusammensetzungen, enthaltend einen blockierten Härtungskatalysator, unter Verwendung von Wärme.**

(30) Priorität : **01.07.83 CH 3636/83**

(43) Veröffentlichungstag der Anmeldung : **23.01.85 Patentblatt 85/04**

(45) Bekanntmachung des Hinweises auf die Patenterteilung : **19.08.87 Patentblatt 87/34**

(84) Benannte Vertragsstaaten : **BE DE FR GB IT NL**

(56) Entgegenhaltungen :
**DE-A- 2 736 231**
**US-A- 3 474 054**

(73) Patentinhaber : **CIBA-GEIGY AG**
**Klybeckstrasse 141**
**CH-4002 Basel (CH)**

(72) Erfinder : **Berner, Godwin, Dr.**
**Waldhofstrasse 70**
**CH-4310 Rheinfelden (CH)**
Erfinder : **Rutsch, Werner, Dr.**
**Avenue Weck-Reynold 1**
**CH-1700 Fribourg (CH)**

**EP 0 132 225 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren, in dem die säurehärtbare Zusammensetzung, enthaltend einen latenten Härtungskatalysator, thermisch gehärtet wird, sowie einige dieser latenten Härtungskatalysatoren und deren Zusammensetzungen.

Es ist bekannt, dass säurehärtbare Harze hauptsächlisch als Bindemittel in Lacken, Druckfarben und Anstrichstoffen verwendet werden. Da die meisten der Säuren, die in Verbindung mit dem Harz als Härtungskatalysatoren dienen, schon bei Raumtemperatur eine langsame Härtung hervorrufen, werden sie dem Harz (oder der jeweiligen entsprechenden Zusammensetzung) erst kurz vor seiner Anwendung hinzugefügt.

Um diesen Nachteil zu vermeiden, wurde Einkomponentensysteme vorgeschlagen, bei denen ein maskierter Härtungskatalysator schon in der säurehärtbaren Zusammensetzung vorhanden ist. Aus diesem kann man die zur Härtung notwendige Säure einerseits durch Bestrahlung mit kurzwelligem Licht und anschliessendem Erwärmen oder andererseits allein durch Erwärmen freisetzen.

Beispiele für latente Härtungskatalysatoren, die mit dem Verfahren « Bestrahlung mit kurzwelligem Licht, anschliessende Erwärmung » aktiviert werden, sind die in der DE-OS 1 919 678 beschriebenen Sulfonsäureester.

Beispiele für latente Härtungskatalysatoren, die durch Erwärmen aktiviert werden, sind Aminsalze von aromatischen Sulfonsäuren, wie die in der Patentschrift U.S. 3 474 054 vorgeschlagenen Pyridinsalze, die jedoch die Nachteile haben können, dass sie bereits während der Lagerung eine langsame Härtung bewirken, dass Geruchsprobleme auftreten und dass kein problemloses elektrostatisches Verspritzen gewährleistet ist. Ausserdem sind Sulfonatester als Katalysatoren in säurehärtbaren Zusammensetzungen, wie in der Patentschrift U.S. 4 281 075 beschrieben, bekannt. Diese Verbindungen genügen jedoch nicht in jeder Hinsicht den zu stellenden Anforderungen, wie z. B. gute Lagerstabilität, sowie eine hohe Freisetzungsrate bei thermischer Behandlung.

Es wurde nun gefunden, dass α-Sulfonyloxycarbonylverbindungen, die technisch einfach herstellbar sind, diese Anforderungen weitgehend erfüllen. Weiterhin führen sie nach der durch Wärme aktivierten Härtung kaum zur Vergilbung. Ueberraschend ist, dass die im erfindungsgemässen Verfahren als latente Härtungskatalysatoren benutzten Verbindungen eine hohe Freisetzungsrate der Sulfonsäure schon bei relativ niedrigen Temperaturen aufweisen. Während unterhalb und bei 100 °C nur eine unbefriedigende Härtung zu erzielen ist, eignen sich die erfindungsgemäss zu verwendenden Katalysatoren bei Temperaturen ab 110 °C ausgezeichnet zur thermischen Härtung von säurehärtbaren Zusammensetzungen.

Die vorliegende Erfindung betrifft ein Verfahren, in dem eine säurehärtbare Zusammensetzung, enthaltend wenigstens einen latenten Härtungskatalysatoer der Formel (I) oder (II)

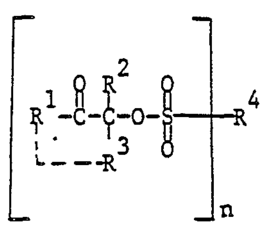

(I)

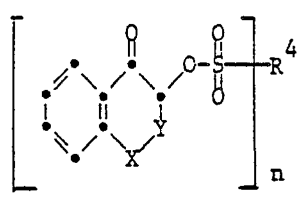

(II)

worin in beiden Formeln n gleich 1 oder 2 ist und

$R^1$ unsubstituiertes oder durch —Cl, —Br, $C_1$-$C_{12}$-Alkyl, $C_1$-$C_4$-Alkoxy, Phenyl oder Phenoxy ein- bis dreifach substituiertes Phenyl, unsubstituiertes oder durch —Cl, —Br, $C_1$-$C_{12}$-Alkyl ein- bis dreifach substituiertes Naphthyl, unsubstituiertes oder durch ein oder mehrere —OR, —Cl, —Br, Phenyl, Cycloalkyl substituiertes $C_1$-$C_{12}$-Alkyl ist, wobei R Wasserstoff, $C_1$-$C_4$-Alkyl oder —$SO_2$—$R^4$ mit dem unten definierten $R^4$ darstellt, oder worin $R^1$ unsubstituiertes oder durch Phenyl substituiertes $C_1$-$C_{12}$-Alkenyl bedeutet

oder Wasserstoff, —OH, $C_1$-$C_4$-Alkoxy, Phenoxy, $C_5$-$C_7$-Cycloalkyl ist oder —$NH_2$, —$NHR^5$, —$N(R^5)_2$, —NH—CO—$R^5$ ist, wobei $R^5$ $C_1$-$C_4$-Alkyl oder Phenyl bedeutet, oder worin $R^1$ Morpholinyl, Piperidinyl, Pyridyl, Furyl, Thienyl, Tetrahydrofuranyl, Tetrahydronaphthyl oder Indolyl ist, und

$R^2$ Wasserstoff bedeutet, und

$R^3$ Wasserstoff, unsubstituiertes oder durch ein oder mehrere —OR, —Cl, —CN, —COOH, $C_2$-$C_5$-Alkoxycarbonyl, Phenyl, Chlorphenyl, $C_1$-$C_{10}$-Alkylphenyl oder $C_1$-$C_{10}$-Alkoxyphenyl substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^3$ unsubstituiertes oder durch ein oder mehrere —Cl, —Br, $C_1$-$C_{12}$-Alkyl, $C_1$-$C_4$-Alkoxy, Phenyl oder Phenoxy substituiertes Phenyl ist oder —CN, —COOH, $C_2$-$C_9$-Alkoxycarbonyl, Benzoyl, —$CONH_2$, —$CONHR^5$, $CON(R^5)_2$,

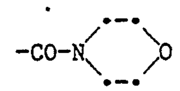 oder 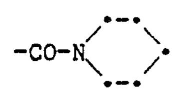

ist, wobei $R^5$ $C_1$-$C_4$-Alkyl oder Phenyl bedeutet, oder

$R^1$ und $R^3$ zusammen mit den C-Atomen, an die sie gebunden sind, einen $C_5$-$C_7$-Cycloalkylring bilden, und

X —O—, —S—, —$SO_2$—, —$CH_2$—, —$C(CH_3)_2$— oder

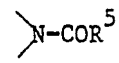,

wobei $R^5$ $C_1$-$C_4$-Alkyl oder Phenyl darstellt, und

Y eine direkte Bindung oder —$CH_2$— bedeutet, und

$R^4$ wenn n = 1 ;

$C_1$-$C_{18}$-Alkyl, unsubstituiertes oder durch Halogen, $C_1$-$C_{18}$-Alkyl, $C_1$-$C_4$-Alkoxy, —NHCO-($C_1$-$C_4$-Alkyl), —NHCO—$C_6H_5$, —$NO_2$ oder Benzoyl ein- bis dreifach substituiertes Phenyl, unsubstituiertes oder durch Halogen, $C_1$-$C_{12}$-Alkyl oder $C_1$-$C_4$-Alkoxy ein- bis dreifach substituiertes Naphthyl, und $R^4$ ausserdem $C_5$-$C_6$-Cycloalkyl, $C_7$-$C_9$-Aralkyl, 10-Campheryl, —$CF_3$, —$CCl_3$ oder —$NH_2$ ist, und

wenn n = 2 :

eine —$(CH_2)_m$-Gruppe, wobei m die Zahl 2 bis 8 darstellt, oder unsubstituiertes oder durch ein oder mehrere $C_1$-$C_{12}$-Alkyl substituiertes Phenylen oder Naphthylen ist,

bei einer Temperatur von 110 °C bis 300 °C thermisch gehärtet wird.

Sind Phenyl oder Naphthyl als $R^1$ durch $C_1$-$C_{12}$-Alkyl substituiert, so handelt es sich um geradkettige oder verzweigte Substituenten, wie beispielsweise Methyl, Aethyl, n-Propyl, Isopropyl, n-Butyl, Isobutyl, sec.-Butyl, tert.-Butyl, geradkettiges oder verzweigtes Pentyl, Hexyl, Heptyl, Octyl, Nonyl, Decyl, Undecyl oder Dodecyl, insbesondere aber um die $C_1$-$C_4$ Alkyldketten. Ist Phenyl als $R^1$ durch $C_1$-$C_4$ Alkoxy substituiert, so handelt es sich beispielsweise um Methoxy, Aethoxy, Propoxy oder tert.-Butoxy, ebenso wenn $R^1$ direkt durch $C_1$-$C_4$-Alkoxy substituiert ist. Handelt es sich bei $R^1$ um $C_1$-$C_{12}$ Alkyl, so liegen geradkettige oder verzweigte Alkylgruppen, wie Methyl, Aethyl, Propyl, Isopropyl, Butyl, tert.-Butyl, Pentyl, Hexyl, Heptyl, Octyl, Nonyl, Decyl, 2-Aethylhexyl, Undecyl oder Dodecyl, vor ; bevorzugt sind die $C_1$-$C_4$ Alkylketten.

Ist $C_1$-$C_{12}$ Alkyl als $R^1$ durch —OH substituiert, so sind alle Monohydroxy-Stellungsisomeren möglich, bevorzugt ist jedoch eine OH-Gruppe am $C_1$- oder $C_2$-Atom der jeweiligen Alkylkette, wie beispielsweise Hydroxymethyl, 1-Hydroxyäthyl, 2-Hydroxyäthyl, 1-Hydroxy-n-propyl, 2-Hydroxy-n-propyl, 1-Hydroxy-isopropyl, 2-Hydroxy-isopropyl oder 1-Hydroxydodecyl. Ist $C_1$-$C_{12}$ Alkyl als $R^1$ durch —Cl oder —Br substituiert, so können bevorzugt 1 bis 4 dieser Halogenatome auftreten, insbesondere sind jedoch die $C_1$- bis $C_4$-Atome der jeweiligen Alkylkette substituiert, wie beispielsweise Monochlormethyl, Monobrommethyl, Dichlormethyl, Trichlormethyl, 1,2-Dichloräthyl, 1,2,3-Trichlorpropyl, 1,2,3,4-Tetrachlorbutyl. Ist $C_1$-$C_{12}$ Alkyl als $R^1$ durch Cycloalkyl substituiert, so handelt es sich um Cyclopentyl oder Cyclohexyl, wobei die jeweiligen Alkylketten nur einmal an ihren $C_1$- bis $C_6$-Atomen substituiert sind, wie Cyclopentylmethyl, 1-Cyclopentyläthyl, 2-Cyclopentyläthyl, Cyclohexylmethyl, 1-Cyclohexyläthyl, 2-Cyclohexyläthyl. Ist $C_1$-$C_{12}$ Alkyl als $R^1$ durch Phenyl substituiert, so ist eine Substitution der Alkylkette durch eine Phenylgruppe in allen Positionen möglich, bevorzugt aber am $C_1$-Atom der jeweiligen Alkylkette, wie beispielsweise Benzyl, α-Methylbenzyl, 1-Phenyl-n-propyl, 1-Phenyl-n-butyl oder α,α-Dimethylbenzyl.

Ist $R^1$ durch $C_1$-$C_{12}$ Alkenyl substituiert, so handelt es sich bevorzugt um eine Doppelbindung in 1,2- oder 3,4-Stellung, wie beispielsweise Aethenyl, n-Propen-(1)-yl, iso-Propen-(1)-yl, n-Buten-(1)-yl, n-Buten-(3)-yl, sec.-Buten-(1)-yl, iso-Buten-(1)-yl, Penten-(1)-yl, Hexen-(1)-yl, Hepten-(1)-yl, Octen-(1)-yl, Nonen-(5)-yl, Decen-(1)-yl, Undecen-(3)-yl, Dodecen-(1)-yl.

Ist $C_1$-$C_{12}$ Alkenyl als $R_1$ durch Phenyl substituiert, so handelt es sich um geradkettige und verzweigte Alkenylketten, die bevorzugt eine Doppelbindung in 1,2- oder 3,4-Stellung und eine Phenylgruppe am $C_2$-Atom der jeweiligen Alkenylketten aufweisen, wie beispielsweise 2-Phenyl-äthenyl, 2-Phenyl-propen-(1)-yl, 2-Phenyl-buten-(1)-yl.

Ist $R^1$ $C_5$-$C_7$ Cycloalkyl, so liegen z. B. Cyclopentyl, Cyclohexyl oder Cycloheptyl, bevorzugt Cyclohexyl, vor.

Bei $R^5$ handelt es sich beispielsweise um Methyl, Aethyl, n-Propyl, n-Butyl, Isopropyl, tert.-Butyl oder Phenyl.

Stellt $R^1$ Thienyl, Pyridyl, Furyl, Indolyl, Tetrahydrofuranyl oder Tetrahydronaphthyl dar, so kommen alle Stellungsisomeren in Betracht. Bevorzugte Stellungsisomeren sind jedoch 2-Thienyl, 3-Pyridyl, 2-Furyl, 3-Indolyl oder 1,2,3,4-Tetrahydro-6-naphthyl.

Bevorzugt ist $R^1$ unsubstituiertes oder substituiertes Phenyl oder Naphthyl, Hydroxy, $C_1$-$C_4$-Alkoxy oder Wasserstoff.

Als $C_1$-$C_8$ Alkyl handelt es sich bei $R^3$ um geradkettige oder verzweigte Alkylgruppen, vorzugsweise aber um geradkettige oder verzweigte $C_4$-$C_8$ Alkylgruppen, wie n-Butyl, sec.-Butyl, tert.-Butyl, Pentyl,

Hexyl, Heptyl oder Octyl.

Sind $C_1$-$C_8$ Alkyl bzw. Phenyl in $R^3$ und Phenyl und Naphthyl in $R^4$ durch $C_1$-$C_4$-Alkoxy substituiert, so handelt es sich beispielsweise um Methoxy-, Aethoxy-, Propoxy- oder tert.-Butoxy-Substituenten.

Ist $C_1$-$C_8$ Alkyl als $R^3$ durch $C_7$-$C_{10}$ Alkylphenyl oder $C_7$-$C_{10}$ Alkoxyphenyl substituiert, so handelt es sich beispielsweise um Methyl-, Methoxy-, Aethyl-, Aethoxy-, tert.-Butyl- oder tert.-Butoxyphenyl-Substituenten, ist es als $R^3$ durch $C_2$-$C_5$ Alkoxycarbonyl substituiert, so handelt es sich z. B. um Methoxy-, Aethoxy-, n-Propoxy-, iso-Propoxy- oder n-Butoxycarbonyl.

Ist Phenyl als $R^3$ durch $C_1$-$C_{12}$-Alkyl substituiert, so liegen z. B. Methyl-, Aethyl, n-Propyl-, iso-Propyl, n-Butyl- oder tert.-Butyl-substituenten vor.

Als $C_2$-$C_9$-Alkoxycarbonyl handelt es sich bei $R^3$ beispielsweise um Methoxy-, Aethoxy-, n-Propoxy-, iso-Propoxy-, n-Butoxy-, Pentoxy-, Hexoxy- oder Heptoxycarbonyl.

Stellt $R^3$ —CO—NHR$^5$ dar, wobei $R^5$ $C_1$-$C_4$-Alkyl ist, und ist $R^4$ durch —NHCO—($C_1$-$C_4$-Alkyl) substituiertes Phenyl, so handelt es sich beispielsweise um Methyl-, Aethyl, Propyl- oder n-Butyl-NHCO- bzw. —CONH-Substituenten.

Sind $C_1$-$C_{12}$-Alkyl in $R^1$ und $C_1$-$C_8$-Alkyl in $R^3$ mehrfach substituiert, so können die Substituenten gleich oder verschieden sein und an das gleiche oder an verschiedene Kohlenstoffatome gebunden sein. Bevorzugt sind 1 bis 3 gleiche oder verschiedene Substituenten, die sich am $C_1$ bis $C_3$ oder am endständigen Kohlenstoffatom in der jeweiligen Alkylkette befinden.

Bilden $R^1$ und $R^3$ zusammen mit den C-Atomen, an die sie gebunden sind, einen Cycloalkylring, so handelt es sich beispielsweise um einen Cyclopentan-, Cyclohexan- oder Cycloheptanring, insbesondere aber um einen Cyclohexanring.

Wenn n = 1 : Stellt $R^4$ $C_1$-$C_{18}$ Alkyl dar, so handelt es sich um geradkettige oder verzweigte Gruppen, wie beispielsweise Methyl, Aethyl, Propyl, Isopropyl, Butyl, tert.-Butyl, Pentyl, Hexyl, Heptyl, Octyl, Nonyl, Decyl, 2-Aethylhexyl, Undecyl, Dodecyl, tert.-Dodecyl, Tri-decyl, Tetradecyl, Hexadecyl oder Octadecyl.

Ist Phenyl bzw. Naphthyl als $R^4$ durch $C_1$-$C_{18}$ Alkyl bzw. durch $C_1$-$C_{12}$-Alkyl substituiert, so handelt es sich um geradkettige oder verzweigte Alkylgruppen, wie z. B. Methyl, Aethyl, n-Propyl, iso-Propyl, n-Butyl, sec.-Butyl, tert.-Butyl, Pentyl, Hexyl, Heptyl, Octyl, 2-Aethylhexyl, Nonyl, Decyl, Undecyl, Dodecyl, 1-Pentylheptyl, Tridecyl, Tetradecyl, Pentadecyl, Hexadecyl, Heptadecyl oder Octadecyl und deren Isomerengemische, insbesondere aber um Isomerengemische mit $C_9$-$C_{15}$-Alkylresten. Ist Phenyl dreifach durch Alkyl substituiert, so handelt es sich um Niederalkylsubstituenten, bevorzugt um Methyl.

Stellt $R^4$ also durch $C_9$-$C_{15}$-Alkyl substituiertes Phenyl dar, so handelt es sich dabei insbesondere um geradkettige Alkylradikale, wobei das Phenyl beispielsweise an dem Nonyl und Decyl in 2-, 3-, 4- oder 5-Stellung, Undecyl und Dodecyl in 2-, 3-, 4-, 5- oder 6-Stellung und an dem Tridecyl und Tetradecyl in 2-, 3-, 4-, 5-, 6- oder 7-Stellung Verknüpfungsstellen aufweisen kann ; es kann sich hierbei auch um Gemische von solchen mit $C_9$-$C_{15}$-Alkyl substituiertem Phenyl handeln.

Stellt $R^4$ $C_5$-$C_6$ Cycloalkyl dar, so handelt, es sich um Cyclopentyl und Cyclohexyl.

Stellt $R^4$ $C_7$-$C_9$ Aralkyl dar, so handelt es sich beispielsweise um 1-Phenyläthyl, 2-Phenyläthyl oder Benzyl.

Wenn n = 2 : Stellt $R^4$ eine —$(CH_2)_m$-Gruppe dar, so handelt es sich beispielsweise um Aethylen, Trimethylen, Tetramethylen, Pentamethylen, Hexamethylen oder Octamethylen.

Sind Phenylen oder Naphthylen durch $C_1$-$C_{12}$ Alkyl substituiert, so handelt es sich dabei um geradkettige oder verzweigte Alkylgruppen, wie z. B. Methyl, Aethyl, n-Propyl, Isopropyl, n-Butyl, sec.-Butyl, tert.-Butyl, Pentyl, Hexyl, Heptyl, Octyl, 2-Aethylhexyl, Nonyl, Decyl, 1-Butylhexyl, Undecyl, Dodecyl, 1-Pentylheptyl.

Sind die verschiedenen Phenylgruppen in den Resten $R^1$, $R^3$ und $R^4$ durch andere Radikale als Wasserstoffatome substituiert, so erfolgt diese Substitution in ortho-, meta- oder para-Stellung, insbesondere aber in para-Stellung.

Sind stereoisomere Formen möglich, so handelt es sich um alle, jedoch bevorzugt um die in der Natur vorkommenden Isomeren.

Beispiele für einzelne Verbindungen der Formeln I und II sind :

a) Formel I mit n = 1 :

2-[(4-Tolylsulfonyl)oxy]-1,2-diphenyl-1-äthanon
2-[(4-Dodecylphenylsulfonyl)oxy]-1,2-diphenyl-1-äthanon
2-[(Phenylsulfonyl)oxy]-1,2-diphenyl-1-äthanon
2-[(Mesitylsulfonyl)oxy]-1,2-diphenyl-1-äthanon
2-[(4-Chlorphenylsulfonyl)oxy]-1,2-diphenyl-1-äthanon
2-[(4-Methoxyphenylsulfonyl)oxy]-1,2-diphenyl-1-äthanon
2-[(4-Acetamidophenylsulfonyl)oxy]-1,2-diphenyl-1-äthanon
2-[(2-Nitrophenylsulfonyl)oxy]-1,2-diphenyl-1-äthanon
2-[(Methylsulfonyl)oxy]-1,2-diphenyl-1-äthanon
2-[(Octylsulfonyl)oxy]-1,2-diphenyl-1-äthanon

2-[(Hexadecylsulfonyl)oxy]-1,2-diphenyl-1-äthanon
2-[(Benzylsulfonyl)oxy]-1,2-diphenyl-1-äthanon
2-[(Cyclohexylsulfonyl)oxy]-1,2-diphenyl-1-äthanon
2-[(Trifluormethylsulfonyl)oxy]-1,2-diphenyl-1-äthanon
2-[(2-Chloräthylsulfonyl)oxy]-1,2-diphenyl-1-äthanon
2-[(2-Naphthylsulfonyl)oxy]-1,2-diphenyl-1-äthanon
2-[(10-Campherylsulfonyl)oxy]-1,2-diphenyl-1-äthanon
2-[(4-Dodecylphenylsulfonyl)oxy]-1,2-bis-(4-methoxyphenyl)-1-äthanon
2-[(4-Dodecylphenylsulfonyl)oxy]-1,2-bis-(4-chlorphenyl)-1-äthanon
2-[(4-Tolylsulfonyl)oxy]-1,2-bis-(4-tolyl)-1-äthanon
2-[(4-Tolylsulfonyl)oxy]-1-(2-naphthyl)-1-äthanon
2-[(4-Tolylsulfonyl)oxy]-essigsäuremethylester
2-[(4-Tolylsulfonyl)oxy]-essigsäure
2-[(Methylsulfonyl)oxy]-propionsäurephenylester
2-[(Phenylsulfonyl)oxy]-essigsäure
2-[(4-Undecylphenylsulfonyl)oxy]-1-äthanal
2-[(4-Tolylsulfonyl)oxy]-N-acetyl-essigsäureamid
1-[(4-Dodecylphenylsulfonyl)oxy]-2-propanon
2-[(4-Tolylsulfonyl)oxy]-bernsteinsäuredimethylester
2-[(4-Tolylsulfonyl)oxy]-4-methyl-valeriansäure
2-[(4-Tolylsulfonyl)oxy]-malonsäuredimethylester
2-[(4-Tolylsulfonyl)oxy]-propionsäure
2-[(Phenylsulfonyl)oxy]-propionsäurebutylester
2-[(4-Tolylsulfonyl)oxy]-dodecansäure
3-[(4-Tolylsulfonyl)oxy]-2-butanon
2-[(4-Tolylsulfonyl)oxy]-3-pentanon
2-[(4-Dodecylphenylsulfonyl)oxy]-2-phenyl-essigsäure
2-[(2-Tolylsulfonyl)oxy]-butanal
3-[(4-Tolylsulfonyl)oxy]-4-methyl-2-pentanon
1-[(3-Tolylsulfonyl)oxy]-4-methyl-2-pentanon
3-[(4-Tolylsulfonyl)oxy]-4-heptanon
2-[(4-Dodecylphenylsulfonyl)oxy]-1-phenyl-1-äthanon
2-[(4-Dodecylphenylsulfonyl)oxy]-1-(4-chlorphenyl)-1-äthanon
2-[(4-Dodecylphenylsulfonyl)oxy]-1-(4-tolyl)-1-äthanon
4-[(4-Tolylsulfonyl)oxy]-3-octanon
2-[(4-Tolylsulfonyl)oxy]-3-oxo-buttersäureäthylester
2-[(4-Tridecylphenylsulfonyl)oxy]-1-(4-biphenyl)-1-äthanon
2-[(4-Tolylsulfonyl)oxy]-1-(4-methoxyphenyl)-1-propanon
2-[(4-Tolylsulfonyl)oxy]-cyclohexanon
2-[(4-Tolylsulfonyl)oxy]-cyclopentanon
2-[(4-Tolylsulfonyl)oxy]-cyclohepdtanon
2-[(4-Tolylsulfonyl)oxy]-3-oxo-3-phenyl-propionsäureäthylester
1-[(3-Tolylsulfonyl)oxy]-1,3-diphenyl-2-propanon
2-[(4-Dodecylphenylsulfonyl)oxy]-1-(4-dodecylphenyl)-äthanon
2-[(4-Tolylsulfonyl)oxy]-1-(4-dodecylphenyl)-1-äthanon
1-[(4-Dodecylphenylsulfonyl)oxy]-3-hydroxy-2-propanon
2-[(4-Tolylsulfonyl)oxy]-1,3-diphenyl-1,3-propandion
2-[((4-(1-Pentylheptyl)phenyl)sulfonyl)oxy]-1,2-diphenyl-1-äthanon
2-[((2,5-Dichlorphenyl)sulfonyl)oxy]-1-(2,4,6-trimethylphenyl)-1-butanon
2-[((2,4,6-Trimethylphenyl)sulfonyl)oxy]-1-(2,4-dichlorphenyl)-1-oxo-hexansäure-äthylester
1-[((4-Methoxyphenyl)sulfonyl)oxy]-5,6-dichlor-6-methyl-2-heptanon
3-[((4-Methoxyphenyl)sulfonyl)oxy]-5,6-dichlor-6-methyl-2-heptanon
2-[((4-Bromphenyl)sulfonyl)oxy]-3-hydroxy-bernsteinsäure-diäthylester
2-[((3-Chlor-4-methyl-phenyl)sulfonyl)oxy]-3-methoxy-1-(4-chlorphenyl)-3-(4-äthoxyphenyl)-1-propanon
2-[((4-(1-Propylhexyl)phenyl)sulfonyl)oxy]-1,2-bis-(3-chlor-4-methyl-phenyl)-1-äthanon
2-[(4-Tolylsulfonyl)oxy]-propionsäure
2-[(4-Tolylsulfonyl)oxy]-butanal
2-[(4-Tolylsulfonyl)oxy]-propionsäure-morpholid
2,3-Bis-[((4-methyl-phenyl)sulfonyl)oxy]-bernsteinsäurediäthylester

b) Formel I mit n = 2

1,4-Butandisulfonsäure-bis-(1,2-diphenyl-2-oxo-äthyl)ester
1,3-Benzoldisulfonsäure-bis-(1,2-diphenyl-2-oxo-äthyl)ester

1,5-Naphthalindisulfonsäure-bis-(2-oxo-2-phenyl-äthyl)ester
Dioctyl-2,6-naphthalindisulfonsäure-bis-(2-oxo-propyl)ester

c) Formel II

2-[(4-Tolylsulfonyl)oxy]-3,4-dihydro-1(2H)-naphthalenon
3-[(4-Dodecylphenylsulfonyl)oxy]-2,3-dihydro-4H-1-benzopyran-4-on
3-(Methylsulfonyloxy)-2,3-dihydro-4H-1-benzothiopyran-4-on
2-[(10-Campherylsulfonyl)oxy]-3(2H)-benzofuranon
2-[(4-Tolylsulfonyl)oxy]-2,3-dihydro-1H-inden-1-on.

Bevorzugt ist ein Verfahren, in welchem eine säurehärtbare Zusammensetzung, enthaltend wenigstens einen Härtungskatalysator der Formel I mit

n = 1, worin

$R^1$ unsubstituiertes oder durch —Cl, —Br oder $C_1$-$C_{12}$-Alkyl substituiertes Phenyl, unsubstituiertes oder durch —Cl, —Br oder $C_1$-$C_{12}$-Alkyl substituiertes Naphthyl, unsubstituiertes oder durch —OR, —Cl oder Phneyl substituiertes $C_1$-$C_{12}$-Alkyl ist, wobei R Wasserstoff, $C_1$-$C_4$-Alkyl oder —$SO_2$—$R^4$ mit dem unten definierten $R^4$ darstellt, oder worin $R^1$ Wasserstoff, —OH oder $C_1$-$C_4$-Alkoxy ist, und

$R^2$ Wasserstoff bedeutet, und

$R^3$ Wasserstoff, unsubstituiertes oder durch —OR, —Cl, —COOH, $C_2$-$C_5$-Alkoxycarbonyl oder Phenyl substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^3$ unsubstituiertes oder durch —Cl, —Br, $C_1$-$C_4$-Alkoxy oder $C_1$-$C_{12}$-Alkyl substituiertes Phenyl ist, und

$R^4$ $C_1$-$C_{18}$-Alkyl, 10-Campheryl, unsubstituiertes oder durch —Cl, —Br, $C_1$-$C_{18}$-Alkyl oder Acetamido substituiertes Phenyl, unsubstituiertes oder durch —Cl, —Br oder $C_1$-$C_{12}$-Alkyl substituiertes Naphthyl bedeutet, thermisch gehärtet wird.

Besonders bevorzugt ist ein Verfahren, in welchem eine säurehärtbare Zusammensetzung, enthaltend wenigstens einen Härtungskatalysator der Formel I mit

n = 1, worin

$R^1$ unsubstituiertes oder durch —Cl, —Br oder $C_1$-$C_4$-Alkyl substituiertes Phenyl, unsubstituiertes oder durch —OR, —Cl oder Phenyl substituiertes $C_1$-$C_{12}$-Alkyl ist, wobei R Wasserstoff, $C_1$-$C_4$-Alkyl oder —$SO_2$—$R^4$ mit dem unten definierten $R^4$ darstellt, oder worin $R^1$ —OH oder $C_1$-$C_4$-Alkoxy ist, und

$R^2$ Wasserstoff bedeutet, und

$R^3$ Wasserstoff, unsubstituiertes oder durch —OR, —COOH, $C_2$-$C_5$-Alkoxycarbonyl oder Phenyl substituiertes $C_1$-$C_8$-Alkyl ist, wobei R Wasserstoff, $C_1$-$C_4$-Alkyl oder —$CO_2$—$R^4$ mit dem unten definierten $R^4$ darstellt, oder worin $R^3$ unsubstituiertes oder durch —Cl, —Br, $C_1$-$C_4$-Alkoxy oder $C_1$-$C_{12}$-Alkyl substituiertes Phenyl ist, und

$R^4$ $C_1$-$C_{18}$-Alkyl, unsubstituiertes oder durch $C_1$-$C_{15}$-Alkyl substituiertes Phenyl, unsubstituiertes oder durch $C_1$-$C_{12}$-Alkyl substituiertes Naphthyl bedeutet, thermisch gehärtet wird.

Insbesondere bevorzugt ist ein Verfahren, in welchem eine säurehärtbare Zusammensetzung, enthaltend wenigstens einen Härtungskatalysator der Formel I mit

n = 1, worin

$R^1$ unsubstituiertes oder durch —Cl oder $C_1$-$C_4$-Alkyl substituiertes Phenyl, unsubstituiertes oder durch —OR substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^1$ —OH oder $C_1$-$C_4$-Alkoxy ist, und

$R^2$ Wasserstoff bedeutet, und

$R^3$ Wasserstoff, unsubstituiertes oder durch —OR oder —COOH substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^3$ unsubstituiertes oder durch —Cl oder $C_1$-$C_{12}$-Alkyl substituiertes Phenyl ist, und

$R^4$ $C_1$-$C_{18}$-Alkyl, unsubstituiertes oder durch $C_1$-$C_{15}$-Alkyl substituiertes Phenyl, unsubstituiertes oder durch $C_1$-$C_{12}$-Alkyl substituiertes Naphthyl bedeutet, thermisch gehärtetet wird.

Speziell bevorzugt ist ein Verfahren, in welchem eine säurehärtbare Zusammensetzung, enthaltend wenigstens einen Härtungskatalysator der Formel I mit

n = 1, worin

$R^1$ unsubstituiertes oder durch Methyl substituiertes Phenyl oder —OH ist, und

$R^2$ Wasserstoff bedeutet, und

$R^3$ Wasserstoff, unsubstituiertes oder durch —OR substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^3$ unsubstituiertes oder durch Methyl substituiertes Phenyl ist, und

$R^4$ Methyl, unsubstituiertes oder durch $C_9$-$C_{15}$-Alkyl oder Methyl substituiertes Phenyl bedeutet, thermisch gehärtet wird.

Ganz speziell bevorzugt ist ein Verfahren, in welchem eine säurehärtbare Zusammensetzung, enthaltend wenigstens einen Härtungskatalysator der Formel I mit n = 1, ausgewählt aus der Gruppe, bestehend aus

2-[(4-Tolylsulfonyl)oxy]-1,2-diphenyl-1-äthanon,
2-[(4-Dodecylsulfonyl)oxy]-1,2-diphenyl-1-äthanon,
2-[(4-Tolylsulfonyl)oxy]-propionsäure,

thermisch gehärtet wird.

Zusätzlich bevorzugt ist ein Verfahren, in welchem eine säurehärtbare Zusammensetzung, enthaltend wenigstens einen Härtungskatalysator der Formel I mit n = 1, worin

$R^1$ unsubstituiertes oder durch —OR, —Cl oder Phenyl substituiertes $C_1$-$C_{12}$-Alkyl ist, wobei R Wasserstoff, $C_1$-$C_4$-Alkyl oder —$SO_2$—$R^4$ mit dem unten definierten $R^4$ darstellt, oder worin $R^1$ —OH oder $C_1$-$C_4$-Alkoxy ist, und

$R^2$ Wasserstoff bedeutet, und

$R^3$ Wasserstoff, unsubstituiertes oder durch —OR, —COOH, $C_2$-$C_5$-Alkoxycarbonyl oder Phenyl substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^3$ unsubstituiertes oder durch —Cl, —Br, $C_1$-$C_4$-Alkoxy oder $C_1$-$C_{12}$-Alkyl substituiertes Phenyl ist, und

$R^4$ $C_1$-$C_{18}$-Alkyl, unsubstituiertes oder durch $C_1$-$C_{15}$-Alkyl substituiertes Phenyl, unsubstituiertes oder durch $C_1$-$C_{12}$-Alkyl substituiertes Naphthyl bedeutet, thermisch gehärtet wird.

Zusätzlich ist insbesondere bevorzugt ein Verfahren, in welchem eine säurehärtbare Zusammensetzung, enthaltend wenigstens einen Härtungskatalysator der Formel I mit

n = 1, worin

$R^1$ unsubstituiertes oder durch —OR substituiertes $C_1$-$C_8$-Alkyl ist, wobei R Wasserstoff, $C_6$-$C_4$-Alkyl oder —$SO_2$—$R^4$ mit dem unten definierten $R^4$ darstellt, oder worin $R^1$ —OH oder $C_1$-$C_4$-Alkoxy ist, und

$R^2$ Wasserstoff bedeutet, und

$R^3$ Wasserstoff, unsubstituiertes oder durch —OR oder —COOH substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^3$ unsubstituiertes oder durch —Cl oder $C_1$-$C_{12}$-Alkyl substituiertes Phenyl ist, und

$R^4$ $C_1$-$C_{18}$-Alkyl, unsubstituiertes oder durch $C_1$-$C_{12}$-Alkyl substituiertes Naphthyl bedeutet, thermisch gehärtet wird.

Einen weiteren Gegenstand der Erfindung stellt eine säurehärtbare Zusammensetzung dar, enthaltend wenigstens einen Härtungskatalysator der Formel I mit n = 1, worin $R^1$ —OH, $R^2$ Wasserstoff, $R^3$ $C_1$-$C_8$-Alkyl und $R^4$ Methyl, Phenyl, Tolyl oder Dodecylphenyl bedeuten ; bevorzugt sind $R^1$ —OH, $R^2$ Wasserstoff, $R^3$ $C_1$-$C_4$-Alkyl und $R^4$ Phenyl oder Tolyl, besonders bevorzugt handelt es sich um den Härtungskatalysator 2-[(4-Tolylsulfonyl)oxy]-propionsäure.

Viele Verbindungen der Formel I sind bekannt und können nach bekannten Verfahren hergestellt werden, beispielsweise durch Umsetzung der entsprechenden Hydroxyverbindungen der Formel III

$$R^1-\overset{\overset{O}{\|}}{C}-\underset{\underset{R^3}{|}}{\overset{\overset{R^2}{|}}{C}}-OH \qquad (III)$$

mit einem bzw. einem halben Aequivalent der entsprechenden Mono- bzw. Di-Sulfonsäurechloride der Formel IV

$$R^4(SO_2Cl)_n \qquad (IV)$$

in Gegenwart einer Base [siehe diesbezüglich : « Journal of the Chemical Society Perkin I, 1981, S. 263 » oder « Journal of Organic Chemistry 34, 1595, (1969) »], oder durch Umsetzung der entsprechenden Bromderivate der Formel V

$$R^1-\overset{\overset{O}{\|}}{C}-\underset{\underset{R^3}{|}}{\overset{\overset{R^2}{|}}{C}}-Br \qquad (V)$$

mit einem bzw. einem halben Aequivalent der Silbersalze der entsprechenden Mono- bzw. Di-Sulfonsäurederivate der Formel VI

$$(AgO_3S)_nR^4 \qquad (VI),$$

wie beispielsweise nach dem in « Journal of Organic Chemistry of the USSR, Band 8, S. 2166 (1972) » angegebenen Verfahren, oder aber für eine bestimmte Klasse dieser Verbindung durch direkte Umsetzung eines Acetophenons der Formel VII mit dem Iodoniumsalz der Formel VIII zum entsprechenden Produkt der Formel IX

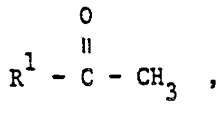 , 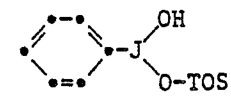 , 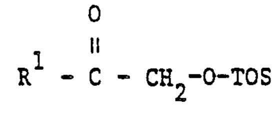 ,

(VII) (VIII) (IX)

(TOS = Tosyl)

wie beispielsweise nach dem in « Journal of Organic Chemistry *47*, 2487 (1982) » angegebenen Verfahren.

In den Formeln III, IV, V und VI, VII und IX haben die Reste $R^1$ bis $R^4$ und n die oben angegebene Bedeutung.

Viele Zwischenprodukte der Formeln III, IV, V, VI und VII stellen bekannte Verbindungen dar, die nach bekannten Verfahren hergestellt werden können, wie z. B. nach denjenigen, die in « Houben-Weyl, Methoden der organischen Chemie » beschrieben sind, Band V/4, S. 171-189, für Verbindungen der Formel V, Band IX, S. 411 bzw. 563, für Verbindungen der Formel IV und für Verbindungen der Formel III, die Literaturstellen « A *526*, 143, (1936), Am. Soc. *76*, 4402 (1954) oder Z. Obsc. Chim. *34*, 3165 (1964) ».

Als säurehärtbare Harze kommen alle Harze in Frage, deren Härtung durch saure Katalysatoren beschleunigt werden kann. Das sind vor allem Lacke auf Basis von Acryl-, Polyester-, Alkyd-, Melamin-, Harnstoff- und Phenolharzen, insbesondere aber die Mischungen von Acryl-, Polyester- oder Alkydharzen untereinander oder mit einem Melaminharz. Darunter fallen auch modifizierte Lackharze, wie z. B. acrylmodifizierte Polyester- oder Alkydharze. Beispiele für einzelne Typen von Harzen, die unter den Begriff Acryl-, Polyester- und Alkydharze fallen, sind z. B. in « Wagner/Sarx : Lackkunstharze, München 1971, S. 86-123, S. 229-238 » oder in « Ullmann, Encyclopädie der technischen Chemie, 4. Auflage, Band 15 (1978), S. 613-628 », EP 44 115, US 4 307 208, US 4 371 605, DE-OS 2 842 002, beschrieben. Von besonderer Bedeutung ist die saure Katalyse für die Härtung von Lacken, die verätherte Amino harze enthalten, wie z. B. methylierte oder butylierte Melaminharze (N-Methoxymethyl- bzw. N-Butoxymethylmelamin oder methylierte/butylierte Glycolurile usw.)

z.B. 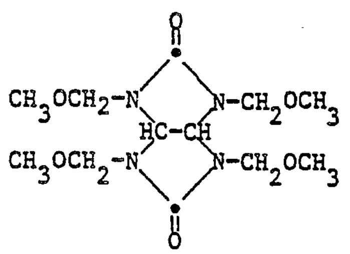

Weitere Harzzusammensetzungen sind Gemische von polyfunktionellen Alkoholen oder Hydroxylgruppen enthaltenden Aryl- und Polyesterharzen, oder partiell verseiftem Polyvinylacetat oder Polyvinylalkohol mit polyfunktionellen Dihydropyranyläthern, wie beispielsweise Derivate der 3,4-Dihydro-2H-pyran-2-carbonsäure.

Für bestimmte Zwecke verwendet man auch Harzzusammensetzungen, die monomere oder oligomere Bestandteile mit polymerisationsfähigen ungesättigten Gruppen haben. Auch solche Harzzusammensetzungen sind nach dem erfindungsgemässen Verfahren härtbar. Hierbei können zusätzlich radikalische Polymerisationsinitiatoren mitverwendet werden ; diese initiieren die Polymerisation der ungesättigten Gruppen während der Wärmebehandlung.

Die Lacke können Lösungen oder Dispersionen des Lackharzes in einem organischen Lösungsmittel oder in Wasser sein, sie können aber auch lösungsmittelfrei sein. Von besonderem Interesse sind Lacke mit geringem Lösungsmittelanteil, sogenannte « high solid »-Lacke. Die Lacke können Klarlacke sein, wie sie z. B. in der Automobilindustrie als Decklacke von Mehrschichten-Anstrichen verwendet werden. Sie können auch Pigmente enthalten, seien es anorganische oder organische Pigmente, sowie Metallpulver für Metalleffekt-Lacke.

Beispiele für polymerisierbare Verbindungen mit einer oder mehreren polymerisierbaren, äthylenisch ungesättigten Bindungen sind Ester der Acryl- und Methacrylsäure, Hydroxyäthylester der Acryl- und Methacrylsäure, Di- und Polyacrylate sowie Di- und Polymethacrylate von Glykolen und Polyolen, aromatische Vinyl- und Divinylderivate, N-Methylolderivate des Acrylamids oder Methacrylamids, Vinylalkyläther, Trimethylolpropandiallyläther-mono-(meth)-acrylate, Reaktionsprodukte von Glycidyl(meth)acrylat und Mono- oder Dicarbonsäuren, Polyesterharze aus $\alpha,\beta$-ungesättigten Dicarbonsäuren oder deren Anhydriden und Diolen, Urethanacrylate oder Polyepoxypolyacrylate.

Die Lacke können weiterhin kleinere Mengen an speziellen Zusätzen enthalten, wie sie in der Lacktechnologie üblich sind, beispielsweise Verlaufsmittel, Thixotropicmittel, Lichtschutzmittel oder Antioxidantien. Beispiele für Lichtschutzmittel sind solche aus der Klasse der Hydroxyphenyl-benztriazole, Hydroxybenzophenone, Cyanacrylate, Hydroxyphenyl-triazine, Oxalanilide, organische Nickelverbindungen oder der Polyalkylpiperidinderivate.

Bevorzugt werden Polymethylpiperidinderivate bzw. Kombinationen derselben mit UV-Absorben verwendet.

Die Härtungskatalysatoren der Formeln I und II werden den Harzen in einer für die Härtung ausreichenden Menge zugesetzt. Die benötigte Menge hängt nicht nur von der Art des Harzes, sondern auch von der beabsichtigten Härtungstemperatur und Härtungszeit ab. Die Härtungszeiten liegen im allgemeinen zwischen 0,1-60 Minuten, bevorzugt zwischen 1-30 Minuten, die Härtungstemperaturen sind allgemein in einem Bereich von 110° bis 300 °C, vorzugsweise aber innerhalb von 120 bis 250 °C und insbesondere 120 bis 150 °C: Ausserdem verwendet man 0,1 bis 10 Gew.-% Härtungskatalysator, vorzugsweise 1-5 Gew.-%, bezogen auf das lösungsmittelfreie Harz. Mischungen der Härtungskatalysatoren können auch umgesetzt werden.

Das vorliegende erfindungsgemässe Verfahren ist für alle Arten der industriellen Oberflächenbeschichtung und Lackierung anwendbar. Darin sind unter anderem die Lackierung von Fahrzeugen, Schiffen, Maschinen und Konstruktionsteilen eingeschlossen. Hervorzuheben ist die Bedeutung, die das erfindungsgemässe Verfahren für die Automobil-Lackierung besitzt, wobei es sowohl für eine Einschichtals auch für eine Mehrschicht-Lackierung verwendbar ist. Besonders wichtig ist, dass dieses Verfahren bei den sogenannten « high solid » Industrielacken eingesetzt werden kann, die — wenn überhaupt — nur geringe Lösungsmittelmengen benötigen, was im Hinblick auf den Umwelt- und Arbeitsschutz (Abluft, Abwasser, Sicherheitseinrichtungen) in Zukunft an Interesse gewinnen wird. Weiterhin ist das Verfahren für die kontinuierliche Beschichtung von Blechen, wie z. B. Stahl- oder Aluminiumbleche, nach dem Coil Coating-Verfahren anwendbar. Ausserdem kann das Verfahren zur Härtung von säurehärtbaren Druckfarben benutzt werden, da diese sich dank ihrer ausgezeichneten Aufziehbarkeit besonders für die Dosenlackierung eignen. Einsetzbar ist dieses Verfahren auch bei Holzbauteilen, die sowohl farblos als auch pigmentiert beschichtet werden.

Zusätzlich zu den bereits genannten Anwendungsmöglichkeiten kann das erfindungsgemässe Verfahren Anwendung auf Pressmassen, Giess- und Laminarharze finden, wobei die Harze in Gegenwart des latenten Härtungskatalysators thermisch aktiviert werden unter gleichzeitiger Verformung und Härtung.

Die folgenden Beispiele erläutern die Herstellung einiger im erfindungsgemässen Verfahren benutzter Verbindungen näher.

## Beispiel 1

Herstellung von 2-[4-(Tolylsulfonyl)oxy]-3-pentanon

Im Kolben werden 19,6 g des Jodoniumsalzes der Formel VIII (0,05 Mol) in 125 ml Acetonitril gelöst und auf Rückfluss (ca. 80 °C) erwärmt. Bei dieser Temperatur werden 50 ml Pentan-(3)-on (0,047 Mol) schnell zugegeben und bei Rückfluss noch 15 Minuten nachgerührt. Die Reaktionslösung wird dann eingeengt, der Rückstand in Methylenchlorid gelöst und mit Wasser gewaschen. Die organische Phase wird getrocknet und erneut eingeengt. Es verbleibt ein klares hellbraunes Oel, welches in der Kälte in Hexan auskristallisiert, wobei ein weisses kristallines Pulver mit einem Schmelzpunkt von 45 °C bis 46 °C in einer Ausbeute von 6,7 g erhalten wird, die 55,6 % der theoretischen entspricht.

Analyse

| | | | | |
|---|---|---|---|---|
| Berechnet | C 56,23 % | H 6,29 % | O 24,97 % | S 12,51 % |
| Gefunden | C 56,47 % | H 6,30 % | O 25,09 % | S 12,48 % |

## Beispiel 2

Herstellung von 2-[(4-Tolylsulfonyl)oxy]-propionsäuremorpholid

In einem Kolben werden 7,5 g (30 mMol) 2-[(4-Tolylsulfonyl)oxy]-propionsäurechlorid in 15 ml Tetrahydrofuran gelöst. Unter Rühren werden dann bei 0-5 °C langsam 6,5 g (80 mMol) Morpholin zugetropft, wobei gleichzeitig gekühlt wird. Darauf wird bei 0 °C nachgerührt, auf Eis gegossen und mit Diäthyläther extrahiert. Die organische Phase wird abgetrennt, mit $Na_2SO_4$ getrocknet und am Vakuumrotationsverdampfer eingeengt. Der hellgelbe Rückstand wird auf einer Kieselgelsäule aufgetrennt (Laufmittel: Hexan / Essigester 1:1): Es resultieren dann weise kristalle mit einem Schmelzpunkt von 75-77 °C.

Elementaranalyse

berechnet : C 53,66 % H 6,11 % N 4,47 % S 10,23 %
gefunden : C 53,57 % H 6,11 % N 4,43 % S 10,29 %

Beispiel 3

Herstellung von 2-[(4-Tolylsulfonyl)oxy]-butanal

In einem Kolben werden 7,2 g (35 mMol) 2-Brombutanal (hergestellt aus Butanal und $CuBr_2$ in Isopropanol/Wasser 72:25) und 12,0 g (42 mMol) Silbertosylat in 100 ml Acetonitril bei 40 °C gerührt. Nach 12 Stunden wird die hellgrüne Suspension filtriert und eingeengt. Der Rückstand wird in 50 ml Methylenchlorid aufgenommen, erneut filtriert und eingeengt. Das Oel wird auf einer Kieselgelsäule aufgetrennt (Laufmittel : Hexan/Essigester 1:1). Es resultiert ein Oel.

Das folgende Beispiel erläutert das erfindungsgemässe Verfahren, in welchem eine säurehärtbare Zusammensetzung, enthaltend einen latenten Härtungskatalysator der Formel I oder II, thermisch gehärtet wird, anhand einer spezifischen Zusammensetzung näher. Hierin bedeuten Teile Gewichtsteile und % Gewichtsprozente.

Beispiel 4

Härtung eines Lackes auf Basis von Acryl-Melamin-Harz.

Auf Aluminium-Bleche von 0,5 mm Dicke, die mit einem Silber-Metallic-Lack als Grundlack auf der Basis von Celluloseacetobutyrat oder Polyester-Melaminharz beschichtet sind, wird ein festkörperreicher Klarlack der folgenden Zusammensetzung aufgetragen :

| | | Festkörper |
|---|---|---|
| Hexamethoxymethylmelamin (Cymel® 301, 100%) | 17,93 g | 17,93 Teile |
| Butylacetat | 9,73 g | |
| Celluloseacetobutyrat (CAB® 551001 der Firma Eastman Chem.) | 1,83 g | |
| Silikonharz in organischem Lösungsmittel (Verlaufshilfsmittel Byketol® Spezial der Firma Byk-Mallinckrodt) | 2,80 g | |
| Verlaufshilfsmittel auf Polymer-Basis (Medaflow®, 1%ige Lsg.; Monsanto) | 0,29 g | |
| Hydroxylfunktionelles Acrylharz (Paraloid® AT 410, 73 Gew.%; Rohm + Haas) | 57,30 g | 41,83 Teile |
| n-Butanol | 10,12 g | |
| | 100,00 g | 59,76 Teile |

Die Katalysatoren werden in einem Gemisch Methylamylketon/Butylglykolacetat im Verhältnis 30 : 70 vorgelöst und dem Lack zugegeben.

Die in Tabelle 1 aufgeführten Verbindungen werden in der Konzentration 1 oder 2 Gew.-% (bezogen auf lösungsmittelfreies Bindemittel = 59,76 Teile) in diese Harzformulierung eingearbeitet.

Der Lack wird mit einem elektrischen Filmziehgerät so aufgetragen, dass die Trockenfilmstärke etwa 45 μm beträgt. Nach einer Abluftzeit von 15 Minuten wird der Lack dann bei 120 °C und 130 °C eingebrannt.

Zur Beurteilung des Härtegrades wird die Pendelhärte des Lackfilms nach der Methode von König (DIN 53 157) bestimmt, und zwar nach 3 Tagen Lagerung.

Zur Beurteilung der Verfärbung (Vergilbung) wird der Farbtonabstand ΔE gemäss DIN 6174 ebenfalls nach 3 Tagen ermittelt.

Ausserdem wird die Lagerstabilität der Lackproben durch Messung der Viskosität mit dem ICI Kegel-Platte-Viskosimeter (DIN 53 229) bestimmt. Dabei wird ein 50 ml Glas mit katalysierter Klarlacklösung

gefüllt (s. Rezeptur ohne Spritzverdünner) und davon die Ausgangsviskosität mit einem ICI-Kegel-Platte-Viskosimeter ermittelt. Die Proben werden dann bei 40 °C oder 60 °C in einem Ofen gelagert, und alle 24 Stunden wird dann die Viskosität gemessen, wobei bestimmt wird, nach wieviel Tagen die Proben geliert sind.

Die Resultate sind aus der folgenden Tabelle 1 zu ersehen.

(Siehe Tabelle 1 Seite 12 f.)

Tabelle 1

| Katalysator | | Einbrenndauer 30 min Einbrenntemperatur 120°C | | Einbrenndauer 30 min Einbrenntemperatur 130°C | | Lagerstabilität bei 40°C | Lagerstabilität bei 60°C |
|---|---|---|---|---|---|---|---|
| Formel | Prozent % | Pendelhärte (s) | Farbtonabstand ΔE | Pendelhärte (s) | Farbtonabstand ΔE | (Tage bis Gelierung) | (Tage bis Gelierung) |
| 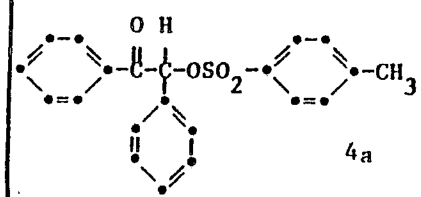 4a | 2 | 134 | 1,1 | 190 | 1,0 | 17 | - |
| 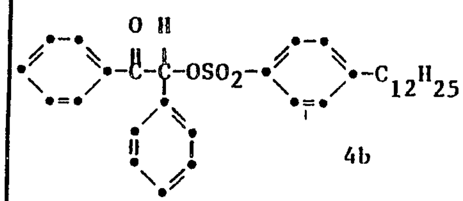 4b | 2 | 119 | 1,2 | 183 | 1,3 | 20 | - |
| 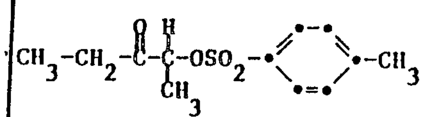 4c | 1 | 165 | 4 | 183 | 4 | - | 7 |

Die in Tabelle 2 gegebenen Resultate sind analog zu den bereits beschriebenen Methoden erhalten worden. Änderungen sind bei der Bestimmung des Härtegrades vorgenommen worden ; die Pendelhärte wird hier nach 24 Stunden gemessen. Ausserdem wird die Lagerstabilität anders ermittelt, nämlich indem der Viskositätsanstieg der Proben nach 7 Tagen gegenüber der Ausgangsviskosität in Prozent angegeben wird.

Tabelle 2

| Katalysator | | Einbrenndauer 30 Min. Einbrenntemp. 120°C | | Einbrenndauer 30 Min. Einbrenntemp. 130°C | | Lagerstabilität (Viskositätszunahme nach 7 Tagen Lagerung bei 40°C; in % gegenüber Ausgangsviskosität) |
|---|---|---|---|---|---|---|
| | Prozent % | Pendelhärte (s) | Farbtonabstand ΔE | Pendelhärte (s) | Farbtonabstand ΔE | |
| ohne Katalysatorzusatz | | 18 | 0,3 | 23 | 0,4 | |
| p-Toluolsulfonsäure | 1 | 189 | 0 | 203 | 0 | bereits nach 1 Tag geliert |
| 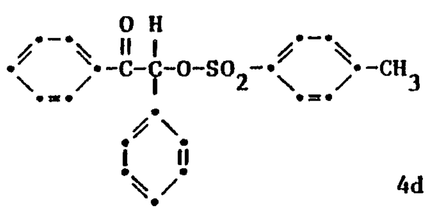 4d | 1<br>2 | 165<br>178 | 0,3<br>0,1 | 183<br>180 | 0,1<br>0,1 | 53<br>107 |
| 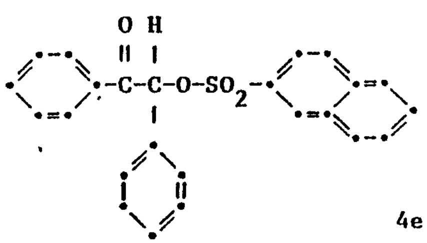 4e | 1<br>2 | 166<br>192 | 0<br>0 | 176<br>196 | 0,3<br>0,2 | 74<br>147 |

Tabelle 2 (Fortsetzung)

| Katalysator | Prozent % | Einbrenndauer 30 Min. Einbrenntemp. 120°C Pendelhärte (s) | Farbtonabstand ΔE | Einbrenndauer 30 Min. Einbrenntemp. 130°C Pendelhärte (s) | Farbtonabstand ΔE | Lagerstabilität (Viskositätszunahme nach 7 Tagen Lagerung bei 40°C; in % gegenüber Ausgangsviskosität) |
|---|---|---|---|---|---|---|
| 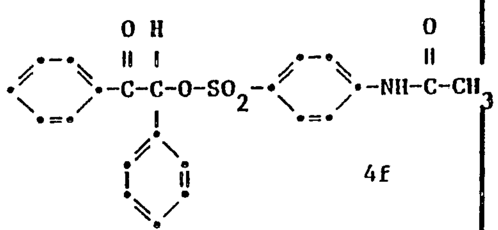 4f | 1 | 178 | 0,2 | 192 | 0,1 | 44 |
| | 2 | 186 | 0 | 204 | 0,1 | -- |
| 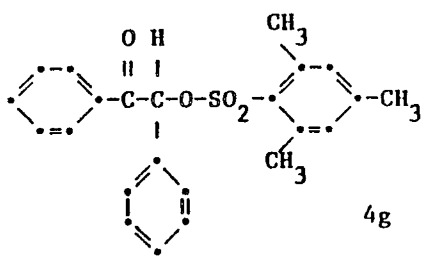 4g | 1 | 85 | 0,1 | 179 | 0,2 | 28 |
| | 2 | 133 | 0,2 | 192 | 0,1 | 61 |
| 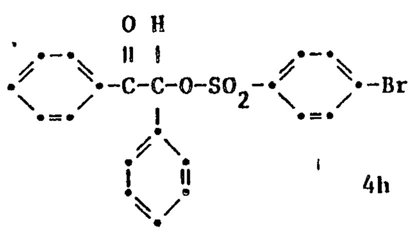 4h | 1 | 193 | 0,1 | 195 | 0,1 | 179 |
| | 2 | 201 | 0,1 | 202 | 0 | 333 |

Tabelle 2 (Fortsetzung)

| Katalysator | | Einbrenndauer 30 Min. Einbrenntemp. 120°C | | Einbrenndauer 30 Min. Einbrenntemp. 130°C | | Lagerstabilität (Viskositätszunahme nach 7 Tagen Lagerung bei 40°C; in % gegenüber Ausgangsviskosität) |
|---|---|---|---|---|---|---|
| | Prozent % | Pendelhärte (s) | Farbtonabstand ΔE | Pendelhärte (s) | Farbtonabstand ΔE | |
| 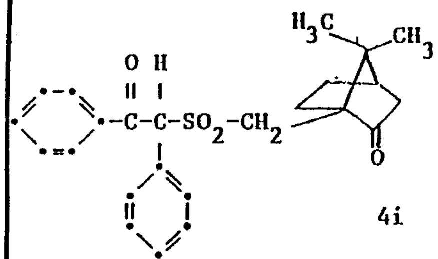 4i | 1<br>2 | 114<br>161 | 0,3<br>0,3 | 190<br>190 | 0,1<br>0,3 | 47<br>71 |
| 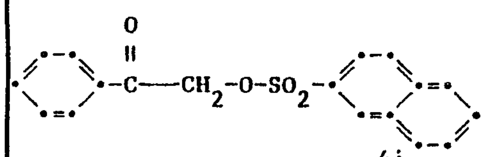 4j | 1<br>2 | 182<br>183 | 0,4<br>0,6 | 200<br>194 | 0,3<br>0,5 | 70<br>106 |
| 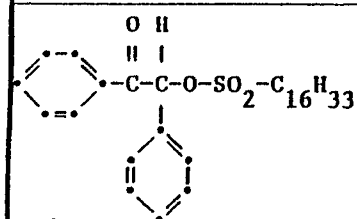 4k | 1 | 195 | 0,2 | 201 | 0 | 135 |
| 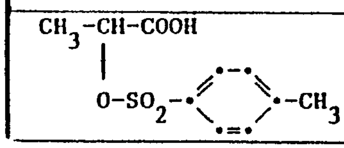 4l | 1 | 202 | 0,1 | 204 | 0,3 | 110 |

Tabelle 2 (Fortsetzung)

| Katalysator | | Einbrenndauer 30 Min. Einbrenntemp. 120°C | | Einbrenndauer 30 Min. Einbrenntemp. 130°C | | Lagerstabilität (Viskositätszunahme nach 7 Tagen Lagerung bei 40°C; in % gegenüber Ausgangsviskosität) |
|---|---|---|---|---|---|---|
| | Prozent % | Pendelhärte (s) | Farbtonabstand ΔE | Pendelhärte (s) | Farbtonabstand ΔE | |
| 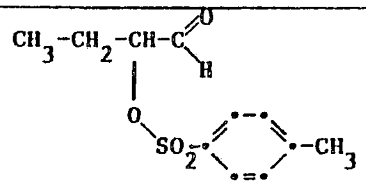 4m | 1 | 96 | 0,3 | 200 | 0,4 | 36 |
| 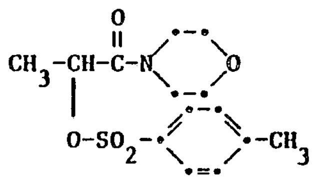 4n | 1<br>2 | 20<br>-- | 0,4<br>--- | 128<br>168 | 0,4<br>0,5 | 14<br>-- |
| 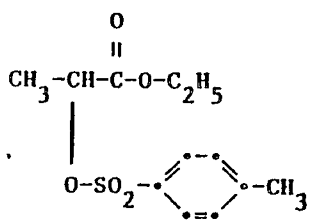 5o | 1<br>2 | 15<br>-- | 0,4<br>--- | 83<br>127 | 0,7<br>0,4 | -- |

**Patentansprüche**

1. Verfahren, in dem eine säurehärtbare Zusammensetzung, enthaltend wenigstens einen latenten Härtungskatalysator der Formel (I) oder (II)

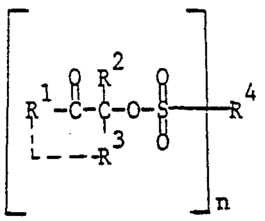

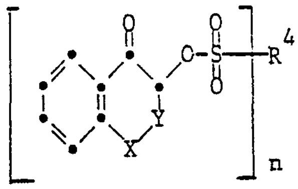

(I) (II)

worin in beiden Formeln

n gleich 1 oder 2 ist und

$R^1$ unsubstituiertes oder durch —Cl, —Br, $C_1$-$C_{12}$-Alkyl, $C_1$-$C_4$-Alkoxy, Phenyl oder Phenoxy ein- bis dreifach substituiertes Phenyl, unsubstituiertes oder durch —Cl, —Br, $C_1$-$C_{12}$-Alkyl ein- bis dreifach substituiertes Naphthyl, unsubstituiertes oder durch ein oder mehrere —OR, —Cl, —Br, Phenyl, Cycloalkyl substituiertes $C_1$-$C_{12}$-Alkyl ist, wobei R Wasserstoff, $C_1$-$C_4$-Alkyl oder —$SO_2$—$R^4$ mit dem unten definierten $R^4$ darstellt, oder worin $R^1$ unsubstituiertes oder durch Phenyl substituiertes $C_1$-$C_{12}$-Alkenyl bedeutet oder Wasserstoff, —OH, $C_1$-$C_4$-Alkoxy, Phenoxy, $C_5$-$C_7$-Cycloalkyl ist oder —$NH_2$, —$NHR^5$, —$N(R^5)_2$, —NH—CO—$R^5$ ist, wobei $R^5$ $C_1$-$C_4$-Alkyl oder Phenyl bedeutet, oder worin $R^1$ Morpholinyl, Piperidinyl, Pyridyl, Furyl, Thienyl, Tetrahydrofuranyl, Tetrahydronaphthyl oder Indolyl ist, und

$R^2$ Wasserstoff bedeutet, und

$R^3$ Wasserstoff, unsubstituiertes oder durch ein oder mehrere —OR, —Cl, —CN, —COOH, $C_2$-$C_5$-Alkoxycarbonyl, Phenyl, Chlorphenyl, $C_7$-$C_{10}$-Alkylphenyl oder $C_7$-$C_{10}$-Alkoxyphenyl substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^3$ unsubstituiertes oder durch ein oder mehrere —Cl, —Br, $C_1$-$C_{12}$-Alkyl, $C_1$-$C_4$-Alkoxy, Phenyl oder Phenoxy substituiertes Phenyl ist oder —CN, —COOH, $C_2$-$C_9$-Alkoxycarbonyl, Benzoyl, —$CONH_2$, —$CONHR^5$, $CON(R^5)_2$,

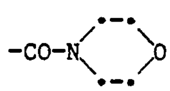 oder 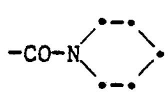

ist,

wobei $R^5$ $C_1$-$C_4$-Alkyl oder Phenyl bedeutet, oder

$R^1$ und $R^3$ zusammen mit den C-Atomen, an die sie gebunden sind, einen $C_5$-$C_7$-Cycloalkylring bilden, und

X —O—, —S—, —$SO_2$—, —$CH_2$—, —$C(CH_3)_2$— oder

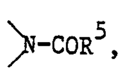,

Alkyl oder Phenyl darstellt, und

Y eine direkte Bindung oder —$CH_2$— bedeutet, und

$R^4$ wenn n = 1 :

$C_1$-$C_{18}$-Alkyl, unsubstituiertes oder durch Halogen, $C_1$-$C_{18}$-Alkyl, $C_1$-$C_4$-Alkoxy, —NHCO—($C_1$-$C_4$-Alkyl), —NHCO—$C_6H_5$, —$NO_2$ oder Benzoyl ein- bis dreifach substituiertes Phenyl, unsubstituiertes oder durch Halogen, $C_1$-$C_{12}$-Alkyl oder $C_1$-$C_4$-Alkoxy ein- bis dreifach substituiertes Naphtyl, und $R^4$ ausserdem $C_5$-$C_6$-Cycloalkyl, $C_7$-$C_9$-Aralkyl, Campheryl, —$CF_3$, —$CCl_3$ oder —$NH_2$ ist, und

wenn n = 2 :

eine —$(CH_2)_m$-Gruppe, wobei m die Zahl 2 bis 8 darstellt, oder unsubstituiertes oder durch ein oder mehrere $C_1$-$C_{12}$-Alkyl substituiertes Phenylen oder Naphthylen ist,

bei einer Temperatur von 110 °C bis 300 °C thermisch gehärtet wird.

2. Ein Verfahren gemäss Anspruch 1, worin in den Härtungskatalysatoren der Formel I

n = 1 ist und

$R^1$ unsubstituiertes oder durch —Cl, —Br oder $C_1$-$C_{12}$-Alkyl substituiertes Phenyl, unsubstituiertes oder durch —Cl, —Br oder $C_1$-$C_{12}$-Alkyl substituiertes Naphthyl, unsubstituiertes oder durch —OR, —Cl oder Phenyl substituiertes $C_1$-$C_{12}$-Alkyl ist, wobei R Wasserstoff, $C_1$-$C_4$-Alkyl oder —$SO_2$—$R^4$ mit dem unten definierten $R^4$ darstellt, worin $R^1$ Wasserstoff, —OH oder $C_1$-$C_4$-Alkoxy ist, und

$R^2$ Wasserstoff bedeutet, und

$R^3$ Wasserstoff, unsubstituiertes oder durch —OR, —Cl, —COOH, $C_2$-$C_5$-Alkoxycarbonyl oder Phenyl substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^3$ unsubstituiertes oder durch —Cl, —Br, $C_1$-$C_4$-Alkoxy oder $C_1$-$C_{12}$-Alkyl substituiertes Phenyl ist, und

$R^4$ $C_1$-$C_{18}$-Alkyl, Campheryl, unsubstituiertes oder durch —Cl, —Br, $C_1$-$C_{18}$-Alkyl oder Acetamido substituiertes Phenyl, unsubstituiertes oder durch —Cl, —Br oder $C_1$-$C_{12}$-Alkyl substituiertes Naphthyl bedeutet.

3. Ein Verfahren gemäss Anspruch 1, worin in den Härtungskatalysatoren der Formel I

n = 1 ist und

$R^1$ unsubstituiertes oder durch —Cl, —Br oder $C_1$-$C_4$-Alkyl substituiertes Phenyl, unsubstituiertes oder durch —OR, —Cl oder Phenyl substituiertes $C_1$-$C_{12}$-Alkyl ist, wobei R Wasserstoff, $C_1$-$C_4$-Alkyl oder —$SO_2$—$R^4$ mit dem unten definierten $R^4$ darstellt, oder worin $R^1$ —OH oder $C_1$-$C_4$-Alkoxy ist, und

$R^2$ Wasserstoff bedeutet, und

$R^3$ Wasserstoff, unsubstituiertes oder durch —OR, —COOH, $C_2$-$C_5$-Alkoxycarbonyl oder Phenyl substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^3$ unsubstituiertes oder durch —Cl, —Br, $C_1$-$C_4$-Alkoxy oder $C_1$-$C_{12}$-Alkyl substituiertes Phenyl ist, und

$R^4$ $C_1$-$C_{18}$-Alkyl, unsubstituiertes oder durch $C_1$-$C_{15}$-Alkyl substituiertes Phenyl, unsubstituiertes oder durch $C_1$-$C_{12}$-Alkyl substituiertes Naphthyl bedeutet.

4. Ein Verfahren gemäss Anspruch I, worin in den Härtungskatalysatoren der Formel I

n = 1 ist und

$R^1$ unsubstituiertes oder durch —Cl oder $C_1$-$C_4$-Alkyl substituiertes Phenyl, unsubstituiertes oder durch —OR substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^1$ —OH oder $C_1$-$C_4$-Alkoxy ist, und

$R^2$ Wasserstoff bedeutet, und

$R^3$ Wasserstoff, unsubstituiertes oder durch —OR oder —COOH substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^3$ unsubstituiertes oder durch —Cl oder $C_1$-$C_{12}$-Alkyl substituiertes Phenyl ist, und

$R^4$ $C_1$-$C_{18}$-Alkyl, unsubstituiertes oder durch $C_1$-$C_{15}$-Alkyl substituiertes Phenyl, unsubstituiertes oder durch $C_1$-$C_{12}$-Alkyl substituiertes Naphthyl bedeutet.

5. Ein Verfahren gemäss Anspruch 1, worin in den Härtungskatalysatoren der Formel I

n = 1 ist und

$R^1$ unsubstituiertes oder durch Methyl substituiertes Phenyl oder —OH ist, und

$R^2$ Wasserstoff bedeutet, und

$R^3$ Wasserstoff, unsubstituiertes oder durch —OR substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^3$ unsubstituiertes oder durch Methyl substituiertes Phenyl ist, und

$R^4$ Methyl, unsubstituiertes oder durch $C_9$-$C_{15}$-Alkyl oder Methyl substituiertes Phenyl bedeutet.

6. Ein Verfahren gemäss Anspruch 1, worin die als latenter Härtungskatalysator eingesetzte Verbindung der Formel I mit n = 1 aus der Gruppe ausgewählt ist, bestehend aus

2-[(4-Tolylsulfonyl)oxy]-1,2-diphenyl-1-äthanon

2-[(4-Dodecylsulfonyl)oxy]-1,2-diphenyl-1-äthanon

2-[(4-Tolylsulfonyl)oxy]-propionsäure.

7. Ein Verfahren gemäss Anspruch 1, worin in den Härtungskatalysatoren der Formel I

n = 1 ist und

$R^1$ unsubstituiertes oder durch —OR, —Cl oder Phenyl substituiertes $C_1$-$C_{12}$-Alkyl ist, wobei R Wasserstoff, $C_1$-$C_4$-Alkyl oder —$SO_2$—$R^4$ mit dem unten definierten $R^4$ darstellt, oder worin $R^1$ —OH oder $C_1$-$C_4$-Alkoxy ist, und

$R^2$ Wasserstoff bedeutet, und

$R^3$ Wasserstoff, unsubstituiertes oder durch —OR, —COOH, $C_2$-$C_5$-Alkoxycarbonyl oder Phenyl substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^3$ unsubstituiertes oder durch —Cl, —Br, $C_1$-$C_4$-Alkoxy oder $C_1$-$C_{12}$-Alkyl substituiertes Phenyl ist, und

$R^4$ $C_1$-$C_{18}$-Alkyl, unsubstituiertes oder durch $C_1$-$C_{15}$-Alkyl substituiertes Phenyl, unsubstituiertes oder durch $C_1$-$C_{12}$-Alkyl substituiertes Naphthyl bedeutet.

8. Ein Verfahren gemäss Anspruch 1, worin in den Härtungskatalysatoren der Formel I

n = 1 ist und

$R^1$ unsubstituiertes oder durch —OR substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^1$ —OH oder $C_1$-$C_4$-Alkoxy ist, und

$R^2$ Wasserstoff bedeutet, und

$R^3$ Wasserstoff, unsubstituiertes oder durch —OR oder —COOH substituiertes $C_1$-$C_8$-Alkyl ist, wobei R Wasserstoff, $C_1$-$C_4$-Alkyl oder —$SO_2$—$R^4$ mit dem unten definierten $R^4$ darstellt, oder worin $R^3$ unsubstituiertes oder durch —Cl oder $C_1$-$C_{12}$-Alkyl substituiertes Phenyl ist, und

$R^4$ $C_1$-$C_{18}$-Alkyl, unsubstituiertes oder durch $C_1$-$C_{15}$-Alkyl substituiertes Phenyl, unsubstituiertes

oder durch $C_1$-$C_{12}$-Alkyl substituiertes Naphthyl bedeutet.

9. Säurehärtbare Zusammensetzung, enthaltend wenigstens einen Härtungskatalysator der Formel I mit n = 1 gemäss Anspruch 1, worin $R^1$ —OH, $R^2$ Wasserstoff, $R^3$ $C_1$-$C_8$-Alkyl und $R^4$ Methyl, Phenyl, Tolyl oder Dodecylphenyl bedeuten.

10. Säurehärtbare Zusammensetzung gemäss Anspruch 9, enthaltend wenigstens einen Härtungskatalysator, worin $R^1$ —OH, $R^2$ Wasserstoff, $R^3$ $C_1$-$C_4$-Alkyl und $R^4$ Phenyl oder Tolyl bedeuten.

11. Säurehärtbare Zusammensetzung gemäss Anspruch 10, enthaltend wenigstens den Härtungskatalysator 2-[(4-Tolylsulfonyl)oxy]-propionsäure.

**Claims**

1. A process in which an acid-curable composition containing at least one latent curing catalyst of formula (I) or (II)

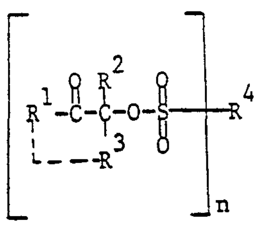

(I)

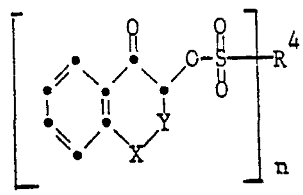

(II)

in which formulae

n is 1 or 2 and

$R^1$ is phenyl or phenyl which is substituted by one, two or three members selected from the group consisting of —Cl, —Br, $C_1$-$C_{12}$alkyl, $C_1$-$C_4$alkoxy, phenyl or phenoxy ; naphthyl or naphthyl which is substituted by one, two or three members selected from the group consisting of —Cl, —Br, and $C_1$-$C_{12}$alkyl ; $C_1$-$C_{12}$alkyl which is unsubstituted or substituted by one or more members selected from the group consisting of —OR, —Cl, —Br, phenyl and cycloalkyl, where R is hydrogen, $C_1$-$C_4$alkyl or —$SO_2$—$R^4$, where $R^4$ is as defined below ; or wherein $R^1$ is unsubstituted or phenyl-substituted $C_1$-$C_{12}$alkenyl ; or is hydrogen, —OH, $C_1$-$C_4$alkoxy, phenoxy or $C_5$-$C_7$cycloalkyl ; or is —$NH_2$, $NHR^5$, —$N(R^5)_2$, —NH—CO—$R^5$, where $R^5$ is $C_1$-$C_4$alkyl or phenyl ; or wherein $R^1$ is morpholinyl, piperidinyl, pyridyl, furyl, thienyl, tetrahydrofuranyl, tetrahydronaphthyl or indolyl ; and

$R^2$ is hydrogen, and

$R^3$ is hydrogen, $C_1$-$C_8$alkyl which is unsubstituted or substituted by one or more members selected from the group consisting of —OR, —Cl, —CN, —COOH, $C_2$-$C_5$alkoxycarbonyl, phenyl, chlorophenyl, $C_7$-$C_{10}$alkylphenyl or $C_7$-$C_{10}$alkoxyphenyl, where R is hydrogen, $C_1$-$C_4$alkyl or —$SO_2$—$R^4$, where $R^4$ is as defined below ; or wherein $R^3$ is phenyl or phenyl which is substituted by one or more members selected from the group consisting of —Cl, —Br, $C_1$-$C_{12}$alkyl, $C_1$-$C_4$alkoxy, phenyl or phenoxy ; or is —CN, —COOH, $C_2$-$C_9$alkoxycarbonyl, benzoyl, —$CONH_2$, —$CONHR^5$, $CON(R^5)_2$,

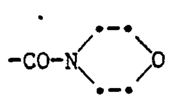 or 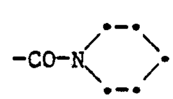

where $R^5$ is $C_1$-$C_4$alkyl or phenyl ; or

$R^1$ and $R^3$, together with the carbon atoms to which they are attached, form a $C_5$-$C_7$cycloalkyl ring, and

X is —O—, —S—, —$SO_2$—, —$CH_2$—, —$C(CH_3)_2$— or

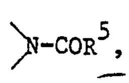,

where $R^5$ is $C_1$-$C_4$alkyl or phenyl, and

Y is a direct bond or —$CH_2$, and

$R^4$ when n = 1, is :

$C_1$-$C_8$alkyl, phenyl or phenyl substituted by one to three members selected from the group consisting of halogen, $C_1$-$C_{18}$alkyl, $C_1$-$C_4$alkoxy, —NHCO($C_1$-$C_4$)alkyl, —NHCO-$C_6H_5$, —$NO_2$ and benzoyl ; or naphthyl or naphthyl substituted by one to three members selected from the group consisting of halogen, $C_1$-$C_{12}$alkyl and $C_1$-$C_4$alkoxy ; and $R^4$ is also $C_5$-$C_6$cycloalkyl, $C_7$-$C_9$aralkyl, camphoryl, —$CF_3$, —$CCl_3$ or —$NH_2$, and,

if n is 2, is:
a $-(CH_2)_m$ group, where m is a number from 2 to 8, or phenylene or naphthylene, each unsubstituted or substituted by one or more $C_1$-$C_{12}$alkyl groups,
is thermally cured in the temperature range from 110° to 300 °C.

2. A process according to claim 1, wherein, in the curing catalysts of the formula I, n = 1 and $R^1$ is phenyl which is unsubstituted or substituted by —Cl, —Br or $C_1$-$C_{12}$alkyl, naphthyl which is unsubstituted or substituted by —Cl, —Br or $C_1$-$C_{12}$alkyl, or $C_1$-$C_{12}$alkyl which is unsubstituted or substituted by —OR, —Cl or phenyl, where R is hydrogen, $C_1$-$C_4$alkyl or $-SO_2-R^4$, where $R^4$ is as defined below, or in which $R^1$ is hydrogen, $C_1$-$C_4$ alkoxy, $R^2$ is hydrogen and $R^3$ is hydrogen, $C_1$-$C_8$alkyl which is unsubstituted or substituted by —OR, —Cl, —COOH, $C_2$-$C_5$alkoxycarbonyl or phenyl, where R is hydrogen, $C_1$-$C_4$alkyl or $-SO_2-R^4$, where $R^4$ is as defined below, or in which $R^3$ is phenyl which is unsubstituted or substituted by —Cl, —Br, $C_1$-$C_4$alkoxy or $C_1$-$C_{12}$alkyl, $R^4$ is $C_1$-$C_{18}$alkyl, camphoryl, phenyl which is unsubstituted or substituted by —Cl, —Br, $C_1$-$C_{18}$alkyl or acetamido, or naphthyl which is unsubstituted or substituted by —Cl, —Br or $C_1$-$C_{12}$alkyl.

3. A process according to claim 1, wherein, in the curing catalysts of the formula I, n is 1 and $R^1$ is phenyl which is unsubstituted or substituted by —Cl, —Br or $C_1$-$C_4$alkyl; $C_1$-$C_{12}$alkyl which is unsubstituted or substituted by —OR, —Cl or phenyl, where R is hydrogen, $C_1$-$C_4$alkyl or $-SO_2-R^4$, where $R^4$ is as defined below, or in which $R^1$ is —OH or $C_1$-$C_4$alkoxy; $R^2$ is hydrogen and $R^3$ is hydrogen, $C_1$-$C_8$alkyl which is unsubstituted or substituted by —OR, —COOH, $C_2$-$C_5$alkoxycarbonyl or phenyl, where R is hydrogen, $C_1$-$C_4$alkyl or $-SO_2-R^4$, where $R^4$ is as defined below, or in which $R^3$ is phenyl which is unsubstituted or substituted by —Cl, —Br, $C_1$-$C_4$alkoxy or $C_1$-$C_{12}$alkyl, and $R^4$ is $C_1$-$C_{18}$alkyl, phenyl which is unsubstituted or substituted by $C_1$-$C_{15}$alkyl, or is naphthyl which is unsubstituted or substituted by $C_1$-$C_{12}$alkyl.

4. A process according to claim 1, wherein, in the curing catalysts of the formula I, n is 1 and $R^1$ is phenyl which is unsubstituted or substituted by —Cl or $C_1$-$C_4$alkyl, or is $C_1$-$C_8$alkyl which is unsubstituted or substituted by —OR, where R is hydrogen, $C_1$-$C_4$alkyl or $-SO_2-R^4$, where $R^4$ is as defined below, or in which $R^1$ is —OH or $C_1$-$C_4$alkoxy; $R^2$ is hydrogen and $R^3$ is hydrogen, $C_1$-$C_8$alkyl which is unsubstituted or substituted by —OR or —COOH, where R is hydrogen, $C_1$-$C_4$alkyl or $-SO_2-R^4$, where $R^4$ is as defined below, or in which $R^3$ is phenyl which is unsubstituted or substituted by —Cl or $C_1$-$C_{12}$alkyl, and $R^4$ is $C_1$-$C_{18}$alkyl, phenyl which is unsubstituted or substituted by $C_1$-$C_{15}$alkyl or naphthyl which is unsubstituted or substituted by $C_1$-$C_{12}$alkyl.

5. A process according to claim 1, wherein, in the curing catalysts of the formula I, n is 1 and $R^1$ is phenyl which is unsubstituted or substituted by methyl, or is —OH, $R^2$ is hydrogen and $R^3$ is hydrogen, $C_1$-$C_8$alkyl which is unsubstituted or substituted by —OR, where R is hydrogen, $C_1$-$C_4$alkyl or $-SO_2-R^4$, where $R^4$ is as defined below, or in which $R^3$ is phenyl which is unsubstituted or substituted by methyl, and $R^4$ is methyl, or phenyl which is unsubstituted or substituted by $C_9$-$C_{15}$alkyl or methyl.

6. A process according to claim 1, wherein the compound of the formula I with n = 1 used as the latent curing catalyst is selected from the group consisting of 2-[(4-tolylsulfonyl)oxy]-1,2-diphenylethan-1-one, 2-[(4-dodecylsulfonyl)oxy]-1,2-diphenylethan-1-one and 2-[(4-tolylsulfonyl)oxy]propionic acid.

7. A process according to claim 1, wherein, in the curing catalysts of the formula I, n is 1 and $R^1$ is $C_1$-$C_{12}$alkyl which is unsubstituted or substituted by —OR, —Cl or phenyl, where R is hydrogen, $C_1$-$C_4$alkyl or $-SO_2-R^4$, where $R^4$ is as defined below, or in which $R^1$ is —OH or $C_1$-$C_4$alkoxy; $R^2$ is hydrogen and $R^3$ is hydrogen, $C_1$-$C_8$alkyl which is unsubstituted or substituted by —OR, —COOH, $C_2$-$C_5$alkoxycarbonyl or phenyl, where R is hydrogen, $C_1$-$C_4$alkyl or $-SO_2-R^4$, where $R^4$ is as defined below, or in which $R^3$ is phenyl which is unsubstituted or substituted by —Cl, —Br, $C_1$-$C_4$alkoxy or $C_1$-$C_{12}$alkyl; and $R^4$ is $C_1$-$C_{18}$alkyl, phenyl which is unsubstituted or substituted by $C_1$-$C_{15}$alkyl, or naphthyl which is unsubstituted or substituted by $C_1$-$C_{12}$alkyl.

8. A process according to claim 1, wherein, in the curing catalysts of the formula I, n is 1 and $R^1$ is $C_1$-$C_8$alkyl which is unsubstituted or substituted by —OR, where R is hydrogen, $C_1$-$C_4$alkyl or $-SO_2-R^4$, where $R^4$ is as defined below, or in which $R^1$ is —OH or $C_1$-$C_4$alkoxy; $R^2$ is hydrogen and $R^3$ is hydrogen, $C_1$-$C_8$alkyl which is unsubstituted or substituted by —OR or —COOH, where R is hydrogen, $C_1$-$C_4$alkyl or $-SO_2-R^4$, where $R^4$ is as defined below, or in which $R^3$ is phenyl which is unsubstituted or substituted by —Cl or $C_1$-$C_{12}$alkyl, and $R^4$ is $C_1$-$C_{18}$alkyl, phenyl which is unsubstituted or substituted by $C_1$-$C_{15}$alkyl, or naphthyl which is unsubstituted or substituted by $C_1$-$C_{12}$alkyl.

9. An acid-curable composition containing at least one curing catalyst of the formula I with n = 1 according to claim 1, in which $R^1$ is —OH, $R^2$ is hydrogen, $R^3$ is $C_1$-$C_8$alkyl and $R^4$ is methyl, phenyl, tolyl or dodecylphenyl.

10. An acid-curable composition according to claim 7, containing at least one hardening catalyst in which $R^1$ is —OH, $R^2$ is hydrogen, $R^3$ is $C_1$-$C_4$alkyl and $R^4$ is phenyl or tolyl.

11. An acid-curable composition according to claim 8, containing at least the curing catalyst 2-[(4-tolylsulfonyl)oxy]propionic acid.

**Revendications**

1. Procédé selon lequel on durcit par la chaleur, à une température de 110 à 300 °C, une composition

durcissable par un acide qui contient au moins un catalyseur de durcissement latent répondant à l'une des formules I et II :

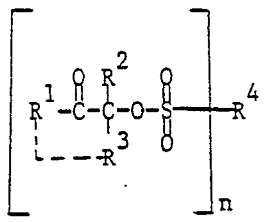

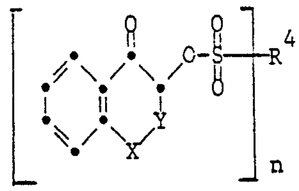

(I) (II)

formules dans lesquelles :

n est égal à 1 ou à 2,

$R^1$ représente un phényle non substitué ou porteur d'1 à 3 substituants pris dans l'ensemble constitué par —Cl, —Br, les alkyles en $C_1$-$C_{12}$, les alcoxy en $C_1$-$C_4$, le phényle et le phénoxy, ou représente un naphtyle non substitué ou porteur d'1 à 3 substituants pris dans l'ensemble constitué par —Cl, —Br et les alkyles en $C_1$-$C_{12}$, ou représente un alkyle en $C_1$-$C_{12}$ non substitué ou porteur d'un ou de plusieurs substituants pris dans l'ensemble constitué par —OR, —Cl, —Br, le phényle et les cycloalkyles, le symbole R désignant l'hydrogène, un alkyle en $C_1$-$C_4$ ou un radical —$SO_2$—$R^4$ dont le symbole $R^4$ est défini ci-dessous, ou représente un alcényle en $C_1$-$C_{12}$ non substitué ou porteur d'un phényle, ou représente l'hydrogène, —OH, un alcoxy en $C_1$-$C_4$, un phénoxy, un cycloalkyle en $C_5$-$C_7$, —$NH_2$, —$NHR^5$, —$N(R^5)_2$ ou —NH—CO—$R^5$, le symbole $R^5$ représentant un alkyle en $C_1$-$C_4$ ou un phényle, ou représente un radical morpholinyle, pipéridyle, pyridyle, furyle, thiényle, tétrahydrofuryle, tétrahydronaphtyle ou indolyle,

$R^2$ représente l'hydrogène,

$R^3$ représente l'hydrogène, ou représente un alkyle en $C_1$-$C_8$ non substitué ou porteur d'un ou plusieurs substituants pris dans l'ensemble constitué par —OR, —Cl, —CN, —COOH, les alcoxycarbonyles en $C_2$-$C_5$, le phényle, les chlorophényles, les alkylphényles en $C_7$-$C_{10}$ et les alcoxyphényles en $C_7$-$C_{10}$, le symbole R représentant l'hydrogène, un alkyle en $C_1$-$C_4$ ou un radical —$SO_2$—$R^4$ dont le symbole $R^4$ est défini ci-dessous, ou représente un phényle non substitué ou porteur d'un ou plusieurs substituants pris dans l'ensemble constitué par —Cl, —Br, les alkyles en $C_1$-$C_{12}$, les alcoxy en $C_1$-$C_4$, le phényle et le phénoxy, ou représente —CN, —COOH, un alcoxycarbonyle en $C_2$-$C_9$, un benzoyle ou un radical —$CONH_2$, —$CONHR^5$, —$CON(R^5)_2$,

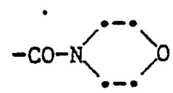 ou 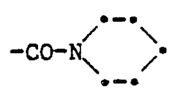

le symbole $R^5$ désignant un alkyle en $C_1$-$C_4$ ou un phényle, ou

$R^1$ et $R^3$ forment ensemble, et avec les atomes de carbone auxquels ils sont liés, un noyau cycloalkylique en $C_5$-$C_7$,

X représente —O—, —S—, —$SO_2$—, —$CH_2$—, —$C(CH_3)_2$— ou

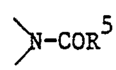,

le symbole $R^5$ désignant un alkyle en $C_1$-$C_4$ ou un phényle,

Y représente une liaison directe ou un radical —$CH_2$— et

$R^4$ représente :

Lorsque n est égal à 1 un alkyle en $C_1$-$C_{18}$, un phényle non substitué ou porteur d'1 à 3 substituants pris dans l'ensemble constitué par les halogènes, les alkyles en $C_1$-$C_{18}$, les alcoxy en $C_1$-$C_4$, les alkylcarbonylamino à alkyle en $C_1$-$C_4$, le phénylcarbonylamino, —$NO_2$ ou le benzoyle, un naphtyle non substitué ou porteur d'1 à 3 substituants pris dans l'ensemble constitué par les halogènes, les alkyles en $C_1$-$C_{12}$ et les alcoxy en $C_1$-$C_4$, ou représente un cycloalkyle en $C_5$ ou $C_6$, un aralkyle en $C_7$-$C_9$, un camphéryle ou un radical —$CF_3$, —$CCl_3$ ou —$NH_2$ et

lorsque n est égal à 2 un radical —$(CH_2)_m$— dont l'indice m désigne un nombre de 2 à 8, ou un radical phénylène ou naphtylène non substitué ou porteur d'un ou plusieurs alkyles en $C_1$-$C_{12}$.

2. Procédé selon la revendication 1 caractérisé en ce que, dans les catalyseurs de durcissement de formule I :

n est égal à 1,

$R^1$ représente un phényle non substitué ou porteur d'un —Cl, d'un —Br ou d'un alkyle en $C_1$-$C_{12}$, un naphtyle non substitué ou porteur d'un —Cl, d'un —Br ou d'un alkyle en $C_1$-$C_{12}$, un alkyle en $C_1$-$C_{12}$ non

substitué ou porteur d'un —OR, d'un —Cl ou d'un phényle, le symbole R désignant l'hydrogène, un alkyle en $C_1$-$C_4$ ou un radical —$SO_2$—$R^4$ dans lequel le symbole $R^4$ a la signification qui est donnée ci-dessous, ou $R^1$ représente l'hydrogène, —OH ou un alcoxy en $C_1$-$C_4$,

$R^2$ représente l'hydrogène,

$R^3$ représente l'hydrogène, un alkyle en $C_1$-$C_8$ non substitué ou porteur d'un radical —OR, —Cl, —COOH, alcoxycarbonyle en $C_2$-$C_5$ ou phényle, R représentant l'hydrogène, un alkyle en $C_1$-$C_4$ ou un radical —$SO_2$—$R^4$ dans lequel $R^4$ a la signification donnée ci-dessous, ou $R^3$ représente un phényle non substitué ou porteur d'un —Cl, d'un —Br, d'un alcoxy en $C_1$-$C_4$ ou d'un alkyle en $C_1$-$C_{12}$, et

$R^4$ représente un alkyle en $C_1$-$C_{18}$, un camphéryle, un phényle non substitué ou porteur d'un —Cl, d'un —Br, d'un alkyle en $C_1$-$C_{18}$ ou d'un acétylamino, ou un naphtyle non substitué ou porteur d'un —Cl, d'un —Br ou d'un alkyle en $C_1$-$C_{12}$.

3. Procédé selon la revendication 1 caractérisé en ce que, dans les catalyseurs de durcissement de formule I :

n est égal à 1,

$R^1$ représente un phényle non substitué ou porteur d'un —Cl, d'un —Br ou d'un alkyle en $C_1$-$C_4$, un alkyle en $C_1$-$C_{12}$ non substitué ou porteur d'un —OR, d'un —Cl ou d'un phényle, R représentant l'hydrogène, un alkyle en $C_1$-$C_4$ ou un radical —$SO_2$—$R^4$ dont le symbole $R^4$ est défini ci-dessous, ou $R^1$ représente un —OH ou un alcoxy en $C_1$-$C_4$,

$R^2$ représente l'hydrogène,

$R^3$ représente l'hydrogène, un alkyle en $C_1$-$C_8$ non substitué ou porteur d'un —OR, d'un —COOH, d'un alcoxycarbonyle en $C_2$-$C_5$ ou d'un phényle, R représentant l'hydrogène, un alkyle en $C_1$-$C_4$ ou un radical —$SO_2$—$R^4$ dans lequel $R^4$ a la signification qui lui est donnée ci-dessous, ou $R^3$ représente un phényle non substitué ou porteur d'un —Cl, d'un —Br, d'un alcoxy en $C_1$-$C_4$ ou d'un alkyle en $C_1$-$C_{12}$, et

$R^4$ représente un alkyle en $C_1$-$C_{18}$, un phényle non substitué ou porteur d'un alkyle en $C_1$-$C_{15}$, ou un naphtyle non substitué ou porteur d'un alkyle en $C_1$-$C_{12}$.

4. Procédé selon la revendication 1 caractérisé en ce que, dans les catalyseurs de durcissement de formule I :

n est égal à 1,

$R^1$ représente un phényle non substitué ou porteur d'un —Cl ou d'un alkyle en $C_1$-$C_4$, un alkyle en $C_1$-$C_8$ non substitué ou porteur d'un —OR, le symbole R désignant l'hydrogène, un alkyle en $C_1$-$C_4$ ou un radical —$SO_2$—$R^4$ dont le symbole $R^4$ est défini ci-dessous, ou $R^1$ représente un —OH ou un alcoxy en $C_1$-$C_4$,

$R^2$ représente l'hydrogène,

$R^3$ représente l'hydrogène, un alkyle en $C_1$-$C_8$ non substitué ou porteur d'un —OR ou d'un —COOH, R représentant l'hydrogène, un alkyle en $C_1$-$C_4$ ou un radical —$SO_2$—$R^4$ dans lequel $R^4$ a la signification indiquée ci-dessous, ou $R^3$ représente un phényle non substitué ou porteur d'un —Cl ou d'un alkyle en $C_1$-$C_{12}$, et

$R^4$ représente un alkyle en $C_1$-$C_{18}$, un phényle non substitué ou porteur d'un alkyle en $C_1$-$C_{15}$, ou un naphtyle non substitué ou porteur d'un alkyle en $C_1$-$C_{12}$.

5. Procédé selon la revendication 1 caractérisé en ce que, dans les catalyseurs de durcissement de formule I :

n est égal à 1,

$R^1$ représente un phényle non substitué ou porteur d'un méthyle ou représente un —OH,

$R^2$ représente l'hydrogène,

$R^3$ représente l'hydrogène, un alkyle en $C_1$-$C_8$ non substitué ou porteur d'un —OR, le symbole R désignant l'hydrogène, un alkyle en $C_1$-$C_4$ ou un radical —$SO_2$—$R^4$ dans lequel $R^4$ a la signification indiquée ci-dessous, ou $R^3$ représente un phényle non substitué ou porteur d'un méthyle, et

$R^4$ représente un méthyle ou représente un phényle non substitué ou porteur d'un alkyle en $C_9$-$C_{15}$ ou d'un méthyle.

6. Procédé selon la revendication 1 caractérisé en ce que le composé de formule I pour lequel n est égal à 1 et qui est utilisé comme catalyseur de durcissement latent est pris dans l'ensemble constitué par :

la (tolyl-4 sulfonyloxy)-2 diphényl-1,2 éthanone-1,
la (dodécyl-4 phénylsulfonyloxy)-2 diphényl-1,2 éthanone-1 et
l'acide (tolyl-4 sulfonyloxy)-2 propionique.

7. Procédé selon la revendication 1 caractérisé en ce que, dans les catalyseurs de durcissement de formule I :

n est égal à 1,

$R^1$ représente un alkyle en $C_1$-$C_{12}$ non substitué ou porteur d'un —OR, d'un —Cl ou d'un phényle, R désignant l'hydrogène, un alkyle en $C_1$-$C_4$ ou un radical —$SO_2$—$R^4$ dont le symbole $R^4$ a la signification indiquée ci-dessous, où $R^1$ représente un —OH ou un alcoxy en $C_1$-$C_4$,

$R^2$ représente l'hydrogène,

$R^3$ représente l'hydrogène, un alkyle en $C_1$-$C_8$ non substitué ou porteur d'un —OR, d'un —COOH, d'un alcoxycarbonyle en $C_2$-$C_5$ ou d'un phényle, R représentant l'hydrogène, un alkyle en $C_1$-$C_4$ ou un

radical $-SO_2-R^4$ dont le symbole $R^4$ a la signification donnée ci-dessous, où $R^3$ représente un phényle non substitué ou porteur d'un —Cl, d'un —Br, d'un alcoxy en $C_1$-$C_4$ ou d'un alkyle en $C_1$-$C_{12}$, et

$R^4$ représente un alkyle en $C_1$-$C_{18}$, un phényle non substitué ou porteur d'un alkyle en $C_1$-$C_{15}$, ou un naphtyle non substitué ou porteur d'un alkyle en $C_1$-$C_{12}$.

8. Procédé selon la revendication 1 caractérisé en ce que, dans les catalyseurs de durcissement de formule I :

n est égal à 1,

$R^1$ représente un alkyle en $C_1$-$C_8$ non substitué ou porteur d'un radical —OR dont le symbole R désigne l'hydrogène, un alkyle en $C_1$-$C_4$ ou un radical $-SO_2-R^4$, le symbole $R^4$ ayant la signification indiquée ci-dessous, ou $R^1$ représente un —OH ou un alcoxy en $C_1$-$C_4$,

$R^2$ représente l'hydrogène,

$R^3$ représente l'hydrogène, un alkyle en $C_1$-$C_8$ non substitué ou porteur d'un —OR ou d'un —COOH, R représentant l'hydrogène, un alkyle en $C_1$-$C_4$ ou un radical $-SO_2-R^4$ dans lequel $R^4$ a la signification indiquée ci-dessous, ou $R^3$ représente un phényle non substitué ou porteur d'un —Cl ou d'un alkyle en $C_1$-$C_{12}$, et

$R^4$ représente un alkyle en $C_1$-$C_{18}$, un phényle non substitué ou porteur d'un alkyle en $C_1$-$C_{15}$, ou un naphtyle non substitué ou porteur d'un alkyle en $C_1$-$C_{12}$.

9. Composition durcissable par un acide, composition qui contient au moins un catalyseur de durcissement de formule I selon la revendication 1 dans lequel n est égal à 1, $R^1$ représente —OH, $R^2$ représente l'hydrogène, $R^3$ représente un alkyle en $C_1$-$C_8$ et $R^4$ représente un radical méthyle, phényle, tolyle ou dodécylphényle.

10. Composition durcissable par un acide, selon la revendication 9, qui contient au moins un catalyseur de durcissement dans lequel $R^1$ représente —OH, $R^2$ représente l'hydrogène, $R^3$ représente un alkyle en $C_1$-$C_4$ et $R^4$ représente un radical phényle ou tolyle.

11. Composition durcissable par un acide, selon la revendication 10, qui contient au moins le catalyseur de durcissement qu'est l'acide (tolyl-4 sulfonyloxy)-2 propionique.